# EUROPEAN PATENT APPLICATION

(11) **EP 4 512 468 A1**
(43) Date of publication of application: **26.02.2025**
(21) Application number: 23791940.2
(22) Date of filing: 21.04.2023
(51) Int. Cl.: A61P 1/16, A61P 1/18, A61P 3/06, A61P 3/10, A61P 9/00, A61P 9/10, A61P 19/10, A61P 27/02, A61P 35/00, A61P 43/00, C07D 213/56, C07D 213/64, A61P 7/06, A61K 31/444, A61P 25/04, A61P 25/16, A61P 25/28, A61P 29/00

(54) **COMPOUND, ALDEHYDE DEHYDROGENASE 2 ACTIVATOR, PHARMACEUTICAL COMPOSITION, AND TREATMENT AND/OR PREVENTATIVE DRUG**

(30) Priority: 22.04.2022 JP 2022070667
(71) Applicant: Alchemedicine, Inc., Tsukuba-shi, Ibaraki 305-0031 (JP)
(72) Inventor: TANAKA, Keigo, Tsukuba-shi, Ibaraki 305-0031 (JP)
(74) Representative: Hoffmann Eitle
(86) International application number: PCT/JP2023/015870
(87) International publication number: WO 2023/204292

(57) **Abstract**

A compound, a pharmaceutically acceptable salt of the compound, or a prodrug of the compound or the salt, the compound represented by the following formula (1): 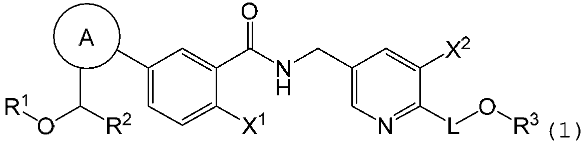 wherein
A is a heterocycle,
R¹ and R² are each independently hydrogen, an alkyl, an alkenyl, or an alkynyl,
R³ is an alkyl, an alkenyl, or an alkynyl,
L is absent or an alkylene,
X¹ is a halogen, and
X² is hydrogen or a halogen.

## Description

### [Technical Field]

The present invention relates to a compound, an aldehyde dehydrogenase 2 activator, a pharmaceutical composition, and a therapeutic and/or prophylactic drug.

### [Background Art]

Aldehyde dehydrogenase 2 (ALDH2) is an enzyme that decomposes aldehydes such as acetaldehyde, and has been reported to have relationship with various diseases (e.g., Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy).

Fanconi's anemia (FA) is a hereditary bone marrow failure disorder, and involves symptoms including aplastic anemia, leukemia, carcinoma, and malformation. It is important for stem cells that produce blood in the bone marrow to properly decompose aldehyde and repair a damaged genome; however, it has been reported that FA patients are incapable of repairing genomic disorder caused by aldehyde, allowing the progression of anemia (e.g., Non Patent Literature 1).

It has been reported with regard to the relationship with osteoporosis that: transgenic mice expressing Aldh2*2 (ALDH2 gene mutation) exhibit symptoms of osteoporosis, which lead to reduction in bone density; the osteoblasts of the model mice show significantly decreased differentiation/formation capability; and osteoblasts with ALDH2 gene mutation show decreased formation capability also in humans (e.g., Non Patent Literature 2).

NAFLD and NASH develop through accumulation of triglyceride in the liver with the background of metabolic disease, and it has been reported that the incidence rate of NAFLD is high in humans having a genotype of low ALDH2 activity (e.g., Non Patent Literature 3). Alcoholic liver disease and pancreatitis are caused by overdrinking, and acetaldehyde generated by decomposition of ethanol in the living body is believed to be the primary cause. In addition, it has been reported that in pathological models of NAFLD, NASH, alcoholic liver disease, and pancreatitis, the pathological condition is ameliorated by an ALDH2 activator compound or introduction of an ALDH2 gene (e.g., Non Patent Literatures 4, 5, and 6).

Ischemia-reperfusion injury is a histological disorder caused by reinitiation of blood supply after continuous ischemic condition due to arterial occlusion in an organ. ALDH2 activator compounds have been reported to be effective for protection against ischemia-reperfusion injury (e.g., Patent Literature 1). In addition, the relationship between peripheral arterial disease and ALDH2 has been suggested (e.g., Non Patent Literature 7).

Alzheimer's disease and Parkinson's disease are cryptogenic neurodegenerative diseases, and it has been reported that drinking and ALDH2 gene mutation contribute to the onset and progression of the pathological condition (e.g., Non Patent Literatures 8 and 9).

Smoking, drinking, and excess intake of hot drinks or foods are believed to be the risk factors of esophageal cancer, and the International Agency for Research on Cancer (IARC) has recognized alcoholic beverages as carcinogens for esophageal squamous cell carcinoma since 2010. Analysis of patients with esophageal cancer and those with head and neck cancer has suggested that alcohol abstinence possibly suppresses the progression of esophageal cancer, and it has been reported that DNA disorder in the esophagus is more strongly caused by drinking in mice with a knocked-in human ALDH2 gene with mutation of low activity; thus, the relationship between esophageal cancer and drinking and ALDH2 has been reported (e.g., Non Patent Literatures 10 and 11). Similarly, the relationship between head and neck cancer and ALDH2 has been reported (e.g., Non Patent Literature 12).

Inflammatory (nociceptive), neuropathic, and cryptogenic pains are mainly known as pain, and it has been reported that in an inflammatory pain model using mice, mice with introduced ALDH2 mutation are more sensitive to pain stimulation, and this tendency is canceled by an ALDH2 activator compound (e.g., Non Patent Literature 13). In addition, it has been reported with a rat model that ALDH2 activation is expected to result in drug efficacy to diabetic retinopathy (e.g., Non Patent Literature 14).

Since relationships between ALDH2 and various diseases have been reported as described above, activation of ALDH2 is expected to be effective for treatment and/or prevention of such diseases. For example, compounds described in Patent Literatures 1 to 5 are known as compounds having activation effect for ALDH2.

### [Citation List]

### [Patent Literature]

[Patent Literature 1]
   International Publication No. WO 2008/112164
[Patent Literature 2]
   International Publication No. WO 2014/160185
[Patent Literature 3]
   International Publication No. WO 2015/127137
[Patent Literature 4]
   International Publication No. WO 2019/151241
[Patent Literature 5]
   Chinese Patent Application Publication No. 108864034

### [Non Patent Literature]

[Non Patent Literature 1]
   Blood (2013) 122 (18): 3206-3209
[Non Patent Literature 2]
   Journal of Bone and Mineral Research (2012) 27 (9): 2015-2023
[Non Patent Literature 3]
   Nutrition & Diabetes (2016) 6, e210
[Non Patent Literature 4]
   Redox Biology (2019) 24: 101205
[Non Patent Literature 5]
   Journal of Hepatology (2015) 62: 647-656
[Non Patent Literature 6]
   Biochemical and Biophysical Research Communications (2020) 522: 518-524
[Non Patent Literature 7]
   Pharmacological Research (2017) 115: 96-106
[Non Patent Literature 8]
   Biochemical and Biophysical Research Communications (2000) 273: 192-196
[Non Patent Literature 9]
   Neurology (2014) 82: 419-426
[Non Patent Literature 10]
   Gastroenterology (2016) 151: 860-869
[Non Patent Literature 11]
   Carcinogenesis (2020) 41: 194-202
[Non Patent Literature 12]
   International Journal of Oncology (2008) 32: 945-973
[Non Patent Literature 13]
   Science Translational Medicine (2014) 6: 251ra118
[Non Patent Literature 14]
   Oxidative Medicine and Cellular Longevity (2021) 2021: ID1641717

### [Summary of Invention]

### [Technical Problem]

An object of the present invention is to provide a compound having ALDH2 activation effect, or an ALDH2 activator, pharmaceutical composition, or therapeutic or prophylactic drug containing the compound.

### [Solution to Problem]

The present inventors diligently examined, and as a result found that compounds of specific structure have ALDH2 activation effect, thus completing the present invention.

The present invention includes the following embodiments.
[1] A compound, a pharmaceutically acceptable salt of the compound, or a prodrug of the compound or the salt, the compound represented by the following formula (1): 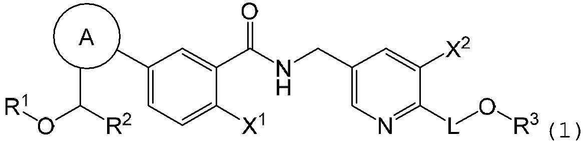 wherein
   A is a heterocycle,
   R¹ and R² are each independently hydrogen, an alkyl, an alkenyl, or an alkynyl,
   R³ is an alkyl, an alkenyl, or an alkynyl,
   L is absent or an alkylene,
   X¹ is a halogen, and
   X² is hydrogen or a halogen.
[2] The compound, pharmaceutically acceptable salt of the compound, or prodrug of the compound or the salt according to [1], wherein A contains one or more nitrogen atoms as a ring member atom or ring member atoms.
[3] The compound, pharmaceutically acceptable salt of the compound, or prodrug of the compound or the salt according to [1] or [2], wherein A is a five- or six-membered ring.
[4] The compound, pharmaceutically acceptable salt of the compound, or prodrug of the compound or the salt according to any one of [1] to [3], wherein A is an aromatic heterocycle.
[5] The compound, pharmaceutically acceptable salt of the compound, or prodrug of the compound or the salt according to any one of [1] to [4], wherein the compound represented by the formula (1) is a compound represented by the following formula (2): 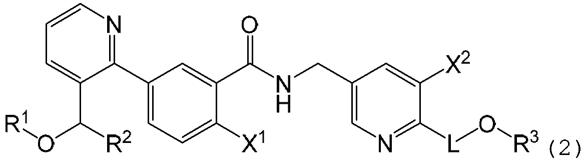 wherein R¹, R², R³, L, X¹, and X² are as described above.
[6A] The compound, pharmaceutically acceptable salt of the compound, or prodrug of the compound or the salt according to any one of [1] to [5], wherein R¹ is hydrogen or an alkyl.
[6B] The compound, pharmaceutically acceptable salt of the compound, or prodrug of the compound or the salt according to any one of [1] to [6A], wherein R¹ is hydrogen.
[7A] The compound, pharmaceutically acceptable salt of the compound, or prodrug of the compound or the salt according to any one of [1] to [6B], wherein R² is hydrogen or an alkyl.
[7B] The compound, pharmaceutically acceptable salt of the compound, or prodrug of the compound or the salt according to any one of [1] to [7A], wherein R² is hydrogen.
[7C] The compound, pharmaceutically acceptable salt of the compound, or prodrug of the compound or the salt according to any one of [1] to [7A], wherein R² is an alkyl.
[8A] The compound, pharmaceutically acceptable salt of the compound, or prodrug of the compound or the salt according to any one of [1] to [7C], wherein R³ is an alkyl.
[8B] The compound, pharmaceutically acceptable salt of the compound, or prodrug of the compound or the salt according to any one of [1] to [8A], wherein R³ is an unsubstituted alkyl, a haloalkyl, or a cycloalkyl.
[8C] The compound, pharmaceutically acceptable salt of the compound, or prodrug of the compound or the salt according to any one of [1] to [8B], wherein R³ is an unsubstituted alkyl.
[8D] The compound, pharmaceutically acceptable salt of the compound, or prodrug of the compound or the salt according to any one of [1] to [8B], wherein R³ is a haloalkyl.
[8E] The compound, pharmaceutically acceptable salt of the compound, or prodrug of the compound or the salt according to any one of [1] to [8B], wherein R³ is a cycloalkyl.
[8F] The compound, pharmaceutically acceptable salt of the compound, or prodrug of the compound or the salt according to any one of [1] to [8B], wherein R³ is methyl, ethyl, isopropyl, cyclopropyl, or fluoromethyl.
[9] The compound, pharmaceutically acceptable salt of the compound, or prodrug of the compound or the salt according to any one of [1] to [8], wherein X¹ is fluorine.
[10] The compound, pharmaceutically acceptable salt of the compound, or prodrug of the compound or the salt according to any one of [1] to [9], wherein X² is hydrogen or fluorine.
[11] The compound, pharmaceutically acceptable salt of the compound, or prodrug of the compound or the salt according to any one of [1] to [10], wherein the compound represented by the formula (1) is selected from the group consisting of the following compounds: 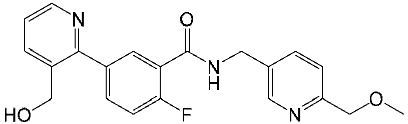 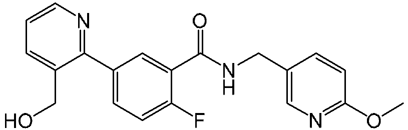 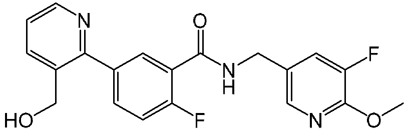 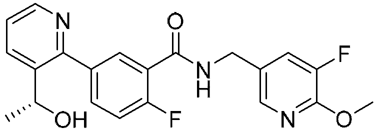 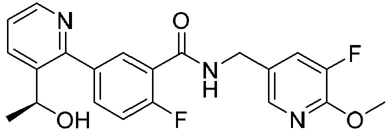 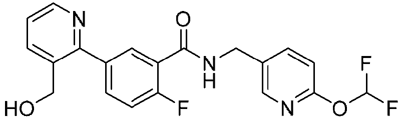 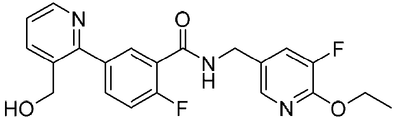 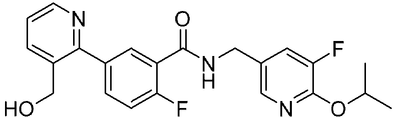 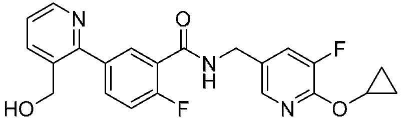
[12] The prodrug according to any one of [1] to [11] or a p harmaceutically acceptable salt of the prodrug, wherein R¹ is -CH₂-O-PO₃H₂.
[13] The prodrug according to any one of [1] to [11] or a pharmaceutically acceptable salt of the prodrug, wherein A contains one or more nitrogen atoms as a ring member atom or ring member atoms, and at least one of the nitrogen atoms is substituted with -CH₂-O-PO₃H₂.
[14] An aldehyde dehydrogenase 2 activator containing the compound, pharmaceutically acceptable salt of the compound, or prodrug of the compound or the salt according to any one of [1] to [13].
[15] A pharmaceutical composition containing the compound, pharmaceutically acceptable salt of the compound, or prodrug of the compound or the salt according to any one of [1] to [13].
[16] A therapeutic and/or prophylactic drug containing the compound, pharmaceutically acceptable salt of the compound, or prodrug of the compound or the salt according to any one of [1] to [13] for a disease selected from the group consisting of Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy.

Moreover, the present invention includes the following embodiments.
[A1] A method for activating aldehyde dehydrogenase 2, the method including administering an effective amount of the compound, pharmaceutically acceptable salt of the compound, or prodrug of the compound or the salt according to any one of [1] to [13] to a patient in need thereof.
[A2] A method for treating and/or preventing a disease selected from the group consisting of Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy, the method including administering an effective amount of the compound, pharmaceutically acceptable salt of the compound, or prodrug of the compound or the salt according to any one of [1] to [13] to a patient in need thereof.
[B1] The compound, pharmaceutically acceptable salt of the compound, or prodrug of the compound or the salt according to any one of [1] to [13] for use for activating aldehyde dehydrogenase 2.
[B2] The compound, pharmaceutically acceptable salt of the compound, or prodrug of the compound or the salt according to any one of [1] to [13] for use for treating and/or preventing a disease selected from the group consisting of Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy.
[C1] Use of the compound, pharmaceutically acceptable salt of the compound, or prodrug of the compound or the salt according to any one of [1] to [13] for activating aldehyde dehydrogenase 2.
[C2] Use of the compound, pharmaceutically acceptable salt of the compound, or prodrug of the compound or the salt according to any one of [1] to [13] for treating and/or preventing a disease selected from the group consisting of Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy.
[D1] Use of the compound, pharmaceutically acceptable salt of the compound, or prodrug of the compound or the salt according to any one of [1] to [14] in production of an aldehyde dehydrogenase 2 activator.
[D2] Use of the compound, pharmaceutically acceptable salt of the compound, or prodrug of the compound or the salt according to any one of [1] to [14] in production of a therapeutic and/or prophylactic drug for a disease selected from the group consisting of Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy.

### [Advantageous Effects of Invention]

The present invention can provide a compound having ALDH2 activation effect, or an ALDH2 activator, pharmaceutical composition, or therapeutic or prophylactic drug containing the compound.

### [Description of Embodiments]

Hereinafter, embodiments of the present invention will be specifically described; however, the present invention is not limited thereto, and various modifications can be made without departing from the spirit of the present invention.

### <Definition>

Herein, the term "comprise (include, contain)" means that not only elements specified to be comprised (included, contained) but also elements other than them may be comprised (included, contained).

### <Compound>

An embodiment of the present invention relates to a compound, a pharmaceutically acceptable salt of the compound, or a prodrug of the compound or the salt, the compound represented by the following formula (1): 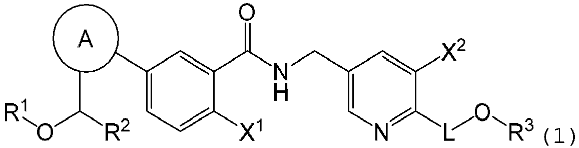 wherein
A is a heterocycle,
R¹ and R² are each independently hydrogen, an alkyl, an alkenyl, or an alkynyl,
R³ is an alkyl, an alkenyl, or an alkynyl,
L is absent or an alkylene,
X¹ is a halogen, and
X² is hydrogen or a halogen.

In the formula (1), it is preferable that A contain one or more nitrogen atoms as a ring member atom or ring member atoms. It is preferable that A be a five- or six-membered ring. It is preferable that A be an aromatic heterocycle. It is preferable that A be a pyridine ring, though A is not limited thereto.

In the formula (1), the alkyl, alkenyl, and alkynyl of R¹ to R³ may be linear or branched or cyclic. The alkyl, alkenyl, and alkynyl of R¹ to R³ may be substituted with a substituent or unsubstituted. Examples of the substituent can include halogens (fluorine, chlorine, bromine, or iodine). If substituents are present, the number of the substituents can be, for example, one, two, or three.

In the formula (1), the alkyls of R¹ to R³ are preferably each independently an alkyl having one to six carbon atoms, more preferably each independently an alkyl having one to three carbon atoms, and further preferably each independently methyl. The alkenyls of R¹ to R³ are preferably each independently an alkenyl having two to six carbon atoms, and more preferably each independently an alkenyl having two to four carbon atoms. The alkynyls of R¹ to R³ are preferably each independently an alkynyl having two to six carbon atoms, and more preferably each independently an alkynyl having two to four carbon atoms.

In the formula (1), R¹ is preferably hydrogen or an alkyl, more preferably hydrogen or methyl, and further preferably hydrogen.

In the formula (1), R² is preferably hydrogen or an alkyl, more preferably hydrogen or methyl, and further preferably hydrogen.

In the formula (1), R³ is preferably an alkyl, more preferably an unsubstituted alkyl, a haloalkyl, or a cycloalkyl, and further preferably methyl, ethyl, isopropyl, cyclopropyl, or fluoromethyl (preferably difluoromethyl).

In the formula (1), L is preferably absent or an alkylene group having one to three carbon atoms, and more preferably absent or methylene (-CH₂-).

In the formula (1), X¹ is preferably fluorine, chlorine, bromine, or iodine, more preferably fluorine or chlorine, and further preferably fluorine.

In the formula (1), X² is preferably hydrogen, fluorine, chlorine, bromine, or iodine, more preferably hydrogen, fluorine or chlorine, and further preferably hydrogen or fluorine.

It is preferable that the compound represented by the formula (1) be a compound represented by the following formula (2): 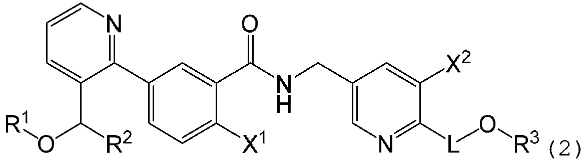 wherein R¹, R², R³, L, X¹, and X² are as described above, though the compound represented by the formula (1) is not limited thereto.

It is preferable that the compound represented by the formula (1) be any of the following compounds: 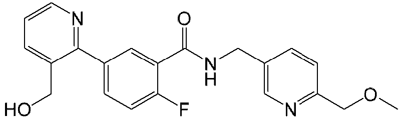 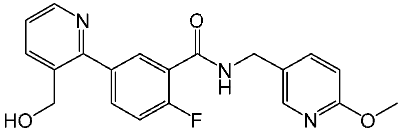 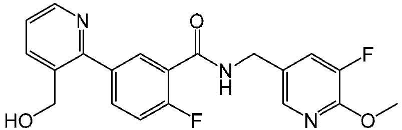 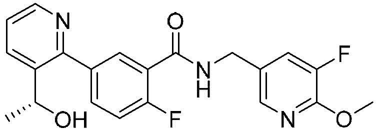 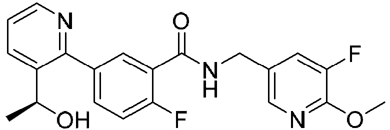 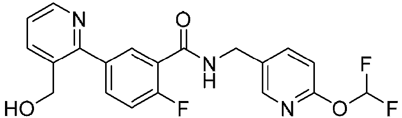 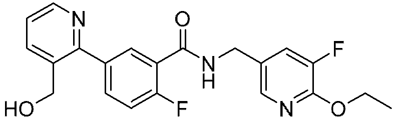 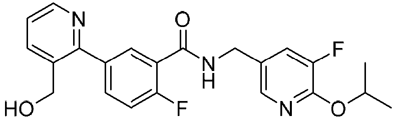 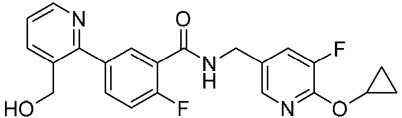 , though the compound represented by the formula (1) is not limited thereto.

Examples of the prodrug of the above compound can include phosphate prodrugs, more specifically, a compound in which R¹ is -CH₂-O-PO₃H₂, and a compound in which at least one of the nitrogen atoms as ring member atoms of A is substituted with -CH₂-O-PO₃H₂.

The pharmaceutically acceptable salt of the above compound or prodrug is not limited and may be any salt that is applicable to medicaments, and examples thereof include inorganic acid salts such as hydrochlorides, sulfates, nitrates, phosphates, and hydrobromides; organic acid salts such as fumarates, maleates, malates, tartrates, succinates, citrates, methanesulfonates, p-toluenesulfonates, acetates, lactates, and palmitates; alkali metal salts; and alkali earth metal salts.

The above compound, pharmaceutically acceptable salt of the compound, or prodrug of the compound or the salt may be forming a solvate such as a hydrate. Herein, such a solvate is encompassed by the scope of the compound, pharmaceutically acceptable salt of the compound, or prodrug of the compound or the salt.

If stereoisomers (e.g., enantiomers, diastereomers) are present for the above compound, pharmaceutically acceptable salt of the compound, or prodrug of the compound or the salt, each of the stereoisomers and any mixture of them (e.g., racemates) are encompassed by the scope of the compound, pharmaceutically acceptable salt of the compound, or prodrug of the compound or the salt.

### <Aldehyde Dehydrogenase 2 Activator>

An embodiment of the present invention relates to an ALDH2 activator containing the above compound, pharmaceutically acceptable salt of the compound, or prodrug of the compound or the salt. The ALDH2 activator of the present embodiment is not only excellent in ALDH2 activation effect, but also further has excellent characteristics (e.g., excellent metabolic stability, prevention of generation of reactive metabolites).

Use of the ALDH2 activator of the present embodiment enables treatment and/or prevention of diseases associated with ALDH2.

### <Pharmaceutical Composition and Therapeutic and/or Prophylactic Drug>

An embodiment of the present invention relates to a pharmaceutical composition containing the above compound, pharmaceutically acceptable salt of the compound, or prodrug of the compound or the salt. An embodiment of the present invention relates to a therapeutic and/or prophylactic drug containing the above compound, pharmaceutically acceptable salt of the compound, or prodrug of the compound or the salt.

Examples of the disease targeted by the pharmaceutical composition and therapeutic and/or prophylactic drug of the present embodiments include Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy.

The pharmaceutical composition and therapeutic and/or prophylactic drug of the present embodiments can be administered orally or parenterally. Examples of dosage forms for oral administration include tablets, pills, granules, powders, capsules, syrups, emulsions, and suspensions. Examples of dosage forms for parenteral administration include injections, infusions, drip infusions, eye drops, and suppositories.

The pharmaceutical composition and therapeutic and/or prophylactic drug of the present embodiments may contain, as necessary, a diluent, a binder, a lubricant, a disintegrator, a sweetening agent, a surfactant, a suspending agent, an emulsifying agent, a coloring agent, a preservative, an aromatic substance, a flavoring agent, a stabilizer, a thickening agent, and so on.

The dose (based on the active ingredient) of the pharmaceutical composition and therapeutic and/or prophylactic drug of the present embodiments varies depending on the condition and body weight of the patient, the type of the compound, the type of the disease, the route of administration, and so on, though physicians can determine an appropriate quantity. In an example for treating Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy, 1 to 2000 (mg) may be administered to an adult (body weight: approximately 60 kg) for oral administration, and 0.01 to 2000 (mg) may be administered to an adult for parenteral administration.

### <Production Method for Compound>

The above compound or pharmaceutically acceptable salt of the compound can be synthesized by appropriately using a known method. The synthesis method can be, in an example, scheme A in the following: 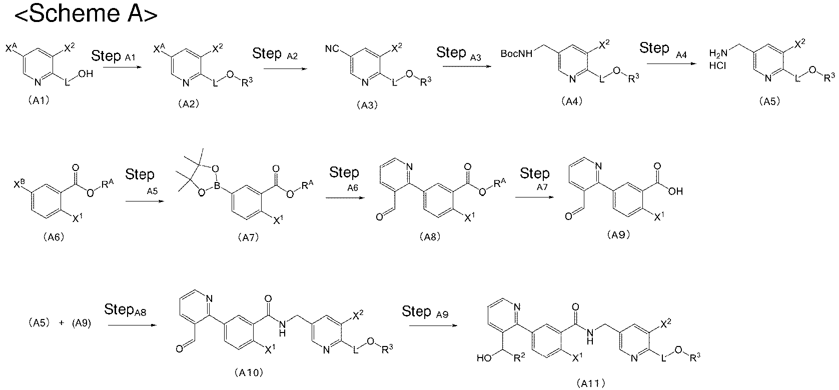 wherein R², R³, L, X¹, and X² are as described above, X^{A} and X^{B} are each independently a halogen, and R^{A} is an alkyl.

The steps of scheme A will be described in the following, whereas those skilled in the art may appropriately set specific conditions in each step on the basis of the contents of Examples shown later.

In step A1, compound (A1) is reacted with a strong base (e.g., sodium hydride), and then reacted with a halide that provides R³ (e.g., iodomethane) to obtain compound (A2).

In step A2, compound (A2) is cyanized with a cyanizing agent (e.g., zinc cyanide) to obtain compound (A3) .

In step A3, the cyano group of compound (A3) is reduced with a reducing agent (e.g., sodium borohydride), and protected with a Boc group to obtain compound (A4).

In step A4, compound (A4) is deprotected to obtain compound (A5).

In step A5, compound (A6) is reacted with bis(pinacolato)diboron to obtain compound (A7).

In step A6, compound (A7) is reacted with 2-bromonicotinaldehyde to obtain compound (A8).

In step A7, the ester group of compound (A8) is hydrolyzed to obtain compound (A9).

In step A8, compound (A5) and compound (A9) are reacted together to obtain compound (A10).

In step A9, compound (A10) is reacted with a reducing agent (e.g., sodium borohydride) or reacted with a Grignard reagent that provides R² to obtain compound (A11).

The synthesis method for the above compound or pharmaceutically acceptable salt of the compound is not limited to scheme A above, and those skilled in the art can appropriately set a suitable synthesis route and reaction conditions according to the structure of the final compound.

The prodrug of the above compound or pharmaceutically acceptable salt of the compound can be suitably synthesized, for example, by introducing a phosphate group with a known method.

If stereoisomers are present for the compound represented by the formula (1), pharmaceutically acceptable salt of the compound, or prodrug of the compound or the salt, the isomers can be resolved with a known method. Examples of such known methods can include chromatography methods, enzymatic methods, and crystallization methods.

### [Examples]

Hereinafter, the present invention will be described in more detail with reference to Examples and Comparative Examples; however, the technical scope of the present invention is not limited thereto.

### [Production Example 1-1]

### (5-Bromopyridin-2-yl)methanol

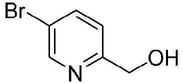

To a mixture of methyl 5-bromopicolinate (5.0 g, 23.2 mmol) and methanol (50 mL), sodium borohydride (1.76 g, 46.5 mmol) was added, and the resultant was stirred at room temperature for 4 hours. Ice-cooled water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (3.20 g).
1H-NMR (400 MHz, CDCl3) δ 8.60 (s, 1H), 7.81 (dd, J=8.4 Hz, J=1.6 Hz, 1H), 7.20 (d, J=8.4 Hz, 1H), 4.71 (s, 2H), 3.36 (bs, 1H).

### [Production Example 1-2]

### 5-Bromo-2-(methoxymethyl)pyridine

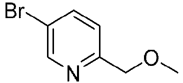

To a mixture of (5-bromopyridin-2-yl)methanol (3.20 g, 17.1 mmol) and DMF (35 mL), 50% sodium hydride (25 g, 0.12 mmol) was slowly added at 0°C, and the resultant was stirred at the same temperature for 30 minutes. To the reaction mixture, iodomethane (3.64 g, 25.6 mmol) was added at room temperature, and the resultant was stirred at room temperature for 3 hours. Ice-cooled water was added to the reaction mixture, which was subjected to extraction with ethyl acetate. The organic layer was dried over sodium sulfate, and the solvent was distilled off under reduced pressure. The residue was purified by silica gel column chromatography (hexane/ethyl acetate) to afford the title compound (2.10 g).
1H-NMR (400 MHz, CDCl3) δ 8.61 (d, J = 2.0 Hz, 1H), 7.83 (dd, J=8.4, 2.4 Hz, 1H), 7.33 (d, J=8.0 Hz, 1H), 4.53 (s, 2H), 3.47 (s, 3H).

### [Production Example 1-3]

### 6-(Methoxymethyl)nicotinonitrile

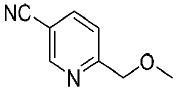

To a mixture of 5-bromo-2-(methoxymethyl)pyridine (1.0 g, 4.97 mmol), zinc (powder, 0.34 g, 5.22 mmol), zinc cyanide (0.87 g, 7.46 mmol), and DMF (10 mL), tris(dibenzylideneacetone)dipalladium(0) (227 mg, 0.24 mmol) and 1,1'-bis(diphenylphosphino)ferrocene (137 mg, 0.24 mmol) were added under an argon atmosphere, and the reaction mixture was stirred at 100°C for 3 hours. The temperature of the reaction mixture was returned to room temperature, water was added thereto, and the resultant was subjected to extraction with ethyl acetate. The organic layer was washed with water and brine and dried over sodium sulfate, and the solvent was distilled off under reduced pressure. The residue was purified by silica gel column chromatography (hexane/ethyl acetate) to afford the title compound (0.45 g).
1H-NMR (400 MHz, CDCl3) δ 8.80 (d, J=1.2 Hz, 1H), 7.97 (dd, J=8.4, 2.4 Hz, 1H), 7.58 (d, J=8.0 Hz, 1H), 4.62 (s, 2H), 3.50 (s, 3H).

### [Production Example 1-4]

### tert-Butyl ((6-(methoxymethyl)pyridin-3-yl)methyl)carbamate

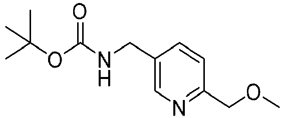

To a mixture of 6-(methoxymethyl)nicotinonitrile (0.50 g, 3.37 mmol) and methanol (10 mL), nickel(II) chloride (1.08 g, 8.44 mmol) and sodium borohydride (0.32 g, 8.44 mmol) were added at 0°C, the resultant was stirred at the same temperature for 15 minutes, di-tert-butyl dicarbonate (2.20 g, 10.0 mmol) was then added thereto, and the resultant was stirred at room temperature for 2 hours. Ice-cooled water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.26 g).
ESI-MS: m/z 253.23 [M+1]+.

### [Production Example 1-5]

### (6-(Methoxymethyl)pyridin-3-yl)methanamine hydrochloride

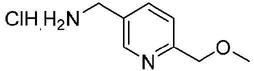

To a mixture of tert-butyl ((6-(methoxymethyl)pyridin-3-yl)methyl)carbamate (0.26 g, 1.03 mmol) and dichloromethane (3.0 mL), 4 M hydrochloric acid-1,4-dioxane solution (1.0 mL) was added at 0°C, and the resultant was stirred at room temperature for 2 hours. The solvent of the reaction mixture was distilled off under reduced pressure to afford the title compound (0.13 g).
ESI-MS: m/z 153.11 [M+1]+.

### [Production Example 1-6]

### Methyl 5-bromo-2-fluorobenzoate

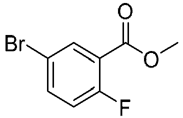

To a mixture of 5-bromo-2-fluorobenzoic acid (5.0 g, 22.8 mmol) and methanol (50 mL), thionyl chloride (8.28 mL, 114 mmol) was slowly added, and the resultant was stirred at 70°C for 8 hours. Methanol in the reaction mixture was distilled off under reduced pressure, and ice-cooled water was added to the residue, which was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (3.80 g).
1H-NMR (400 MHz, CDCl3) δ 8.06 (dd, J=6.4, 2.4 Hz, 1H), 7.59-7.63 (m, 1H), 7.04 (t, J = 9.6 Hz, 1H), 3.93 (s, 3H).

### [Production Example 1-7]

### Methyl 2-fluoro-5-(4,4,5,5-tetramethyl-1,3,2-dioxaborolan-2-yl)benzoate

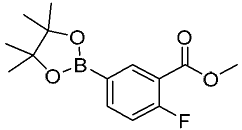

To a mixture of methyl 5-bromo-2-fluorobenzoate (3.80 g, 16.3 mmol), bis(pinacolato)diboron (6.21 g, 24.4 mmol), potassium acetate (3.19 g, 32.6 mmol), and 1,4-dioxane (40 mL), a dichloromethane adduct of dichloro(1,1'-bis(diphenylphosphino)ferrocene)palladium (0.665 g, 0.81 mmol) was added under an argon atmosphere, and the resultant was stirred at 100°C for 6 hours. The temperature of the reaction mixture was returned to room temperature, the reaction mixture was filtered through a Celite, and the solvent of the filtrate was distilled off under reduced pressure. The residue was purified by silica gel column chromatography (hexane/ethyl acetate) to afford the title compound (2.40 g).
ESI-MS: m/z 281.38 [M+1]+.

### [Production Example 1-8]

### Methyl 2-fluoro-5-(3-formylpyridin-2-yl)benzoate

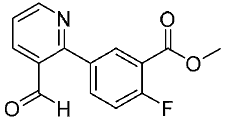

To a mixture of methyl 2-fluoro-5-(4,4,5,5-tetramethyl-1,3,2-dioxaborolan-2-yl)benzoate (2.40 g, 8.57 mmol), 2-bromonicotinaldehyde (1.58 g, 8.57 mmol), potassium carbonate (2.36 g, 17.1 mmol), 1,4-dioxane (24 mL), and water (6 mL), a dichloromethane adduct of dichloro(1,1'-bis(diphenylphosphino)ferrocene)palladium (0.34 g, 0.42 mmol) was added under an argon atmosphere, and the resultant was stirred at 100°C for 15 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (1.10 g).
ESI-MS: m/z 260.28 [M+1]+.

### [Production Example 1-9]

### 2-Fluoro-5-(3-formylpyridin-2-yl)benzoic acid

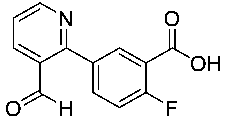

To a mixture of methyl 2-fluoro-5-(3-formylpyridin-2-yl)benzoate (1.10 g, 4.23 mmol), tetrahydrofuran (5 mL), methanol (5 mL), and water (5 mL), lithium hydroxide (0.304 g, 12.6 mmol) was added at 0°C, and the resultant was stirred at room temperature for 4 hours. Ice-cooled water was added to the reaction mixture, which was washed with ethyl acetate. The aqueous layer was neutralized with 1 mol/L hydrochloric acid, and subjected to extraction with ethyl acetate. The organic layer was dried over sodium sulfate, and the solvent was distilled off under reduced pressure to afford the title compound (0.70 g).
ESI-MS: m/z 246.24 [M+1]+.

### [Production Example 1-10]

### 2-Fluoro-5-(3-formylpyridin-2-yl)-N-((6-(methoxymethyl)pyridin-3-yl)methyl)benzamide

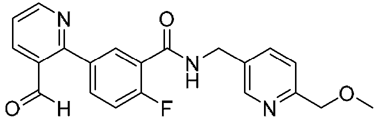

To a mixture of 2-fluoro-5-(3-formylpyridin-2-yl)benzoic acid (0.40 g, 1.63 mmol), (6-(methoxymethyl)pyridin-3-yl)methanamine hydrochloride (0.30 g, 1.63 mmol), N,N-diisopropylethylamine (0.84 g, 6.53 mmol), and dichloromethane (10 mL), 500 propylphosphonic anhydride-ethyl acetate solution (3.10 g, 4.89 mmol) was added, and the reaction mixture was stirred at room temperature for 6 hours. The reaction mixture was diluted with dichloromethane and washed with 1 mol/L hydrochloric acid, water, saturated aqueous solution of sodium hydrogen carbonate, and brine, the organic layer was dried over sodium sulfate, and the solvent was distilled off under reduced pressure. The residue was purified by silica gel column chromatography (hexane/ethyl acetate) to afford the title compound (0.23 g).
ESI-MS: m/z 380.39 [M+1]+.

### [Example 1]

### 2-Fluoro-5-(3-(hydroxymethyl)pyridin-2-yl)-N-((6-(methoxymethyl)pyridin-3-yl)methyl)benzamide

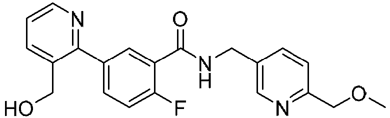

To a mixture of 2-fluoro-5-(3-formylpyridin-2-yl)-N-((6-(methoxymethyl)pyridin-3-yl)methyl)benzamide (0.23 g, 0.60 mmol) and methanol (5.0 mL), sodium borohydride (0.057 g, 1.51 mmol) was added at 0°C, and the resultant was stirred at room temperature for 3 hours. Water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by high-performance liquid chromatography (X-Bridge C18 (19 × 250) 10 µ, 5 mM aqueous solution of ammonium hydrogen carbonate) to afford the title compound (0.042 g). ESI-MS: m/z 382.18 [M+1]+.
1H-NMR (400 MHz, DMSO-d6) δ 9.03 (t, J=5.6 Hz, 1H), 8.56 (dd, J=4.8, 1.2 Hz, 1H), 8.56 (d, J=1.6 Hz, 1H), 7.96 (d, J=7.6 Hz, 1H), 7.86 (dd, J=6.8, 2.0 Hz, 1H), 7.80-7.74 (m, 2H), 7.44-7.37 (m,3H), 5.45 (t, J=5.2 Hz, 1H), 4.50-4.47 (m, 6H), 3.34 (s, 3H).

### [Production Example 2-1]

### 6-Methoxynicotinonitrile

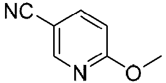

To a mixture of 5-bromo-2-methoxypyridine (5.0 g, 26.7 mmol), zinc (powder, 1.82 g, 28.0 mmol), zinc cyanide (4.70 g, 41.0 mmol), and DMF (10 mL), tris(dibenzylideneacetone)dipalladium(0) (1.22 g, 1.3 mmol) and 1,1'-bis(diphenylphosphino)ferrocene (740 mg, 1.3 mmol) were added under an argon atmosphere, and the reaction mixture was stirred at 110°C for 2 hours. The temperature of the reaction mixture was returned to room temperature, ice-cooled water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (2.40 g).
1H-NMR (400Hz, DMSO-d6) δ 8.49 (d, J=2.0 Hz, 1H), 7.76 (dd, J=8.8, 2.4 Hz, 1H), 6.81 (d, J=8.8 Hz, 1H), 3.91 (s, 3H) .

### [Production Example 2-2]

### tert-Butyl ((6-methoxypyridin-3-yl)methyl)carbamate

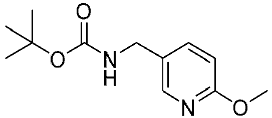

To a mixture of 6-methoxynicotinonitrile (2.40 g, 17.9 mmol) and methanol (30 mL), nickel(II) chloride (5.79 g, 44.7 mmol) and sodium borohydride (1.70 g, 44.7 mmol) were added at 0°C, the resultant was stirred at the same temperature for 15 minutes, di-tert-butyl dicarbonate (11.7 g, 53.7 mmol) was then added thereto, and the resultant was stirred at 0°C for 2 hours. Ice-cooled water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (1.40 g).
ESI-MS: m/z 239.36 [M+1]+.

### [Production Example 2-3]

### (6-Methoxypyridin-3-yl)methanamine hydrochloride

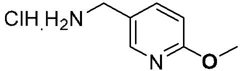

To a mixture of tert-butyl ((6-methoxypyridin-3-yl)methyl)carbamate (1.20 g, 5.04 mmol) and dichloromethane (15.0 mL), 4 M hydrochloric acid-1,4-dioxane solution (10.0 mL) was added at 0°C, and the resultant was stirred at room temperature for 2 hours. The solvent of the reaction mixture was distilled off under reduced pressure to afford the title compound (0.85 g).
1H-NMR (400Hz, DMSO-d6) δ 8.47 (bs, 2H), 8.26 (d, J=2.0 Hz, 1H), 7.87 (dd, J=8.4, 2.0 Hz, 1H), 6.88 (d, J=8.4 Hz, 1H), 3.96 (q, J = 5.6 Hz, 2H), 3.85 (s, 3H).

### [Production Example 2-4]

### 5-Bromo-2-fluoro-N-((6-methoxypyridin-3-yl)methyl)benzamide

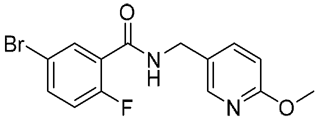

To a mixture of 5-bromo-2-fluorobenzoic acid (1.18 g, 5.43 mmol), (6-methoxypyridin-3-yl)methanamine hydrochloride (0.75 g, 5.43 mmol), N,N-diisopulethylamine (2.79 g, 21.7 mmol), and dichloromethane (20 mL), 500 propylphosphonic anhydride-ethyl acetate solution (10.36 g, 16.2 mmol) was added, and the reaction mixture was stirred at room temperature for 6 hours. The reaction mixture was diluted with dichloromethane and washed with 1 mol/L hydrochloric acid, water, saturated aqueous solution of sodium hydrogen carbonate, and brine, the organic layer was dried over sodium sulfate, and the solvent was distilled off under reduced pressure. The residue was purified by silica gel column chromatography (hexane/ethyl acetate) to afford the title compound (0.60 g).
ESI-MS: m/z 339.25 [M+1]+.

### [Production Example 2-5]

### 2-Fluoro-N-((6-methoxypyridin-3-yl)methyl)-5-(4,4,5,5-tetramethyl-1,3,2-dioxaborolan-2-yl)benzamide

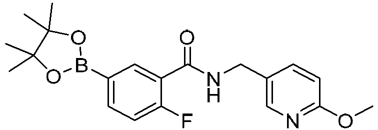

To a mixture of 5-bromo-2-fluoro-N-((6-methoxypyridin-3-yl)methyl)benzamide (0.60 g, 1.77 mmol), bis(pinacolato)diboron (0.67 g, 2.66 mmol), potassium acetate (0.34 g, 3.54 mmol), and 1,4-dioxane (10 mL), a dichloromethane adduct of dichloro(1,1'-bis(diphenylphosphino)ferrocene)palladium (0.071 g, 0.088 mmol) was added under an argon atmosphere, and the resultant was stirred at 100°C for 6 hours. The temperature of the reaction mixture was returned to room temperature, the reaction mixture was filtered through a Celite, and the solvent of the filtrate was distilled off under reduced pressure. The residue was purified by silica gel column chromatography (hexane/ethyl acetate) to afford the title compound (0.55 g).
ESI-MS: m/z 387.32 [M+1]+.

### [Production Example 2-6]

### 2-Fluoro-5-(3-formylpyridin-2-yl)-N-((6-methoxypyridin-3-yl)methyl)benzamide

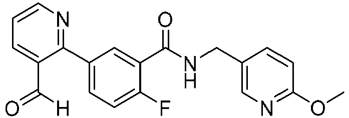

To a mixture of 2-fluoro-N-((6-methoxypyridin-3-yl)methyl)-5-(4,4,5,5-tetramethyl-1,3,2-dioxaborolan-2-yl)benzamide (0.55 g, 1.42 mmol), 2-bromonicotinaldehyde (0.26 g, 1.42 mmol), potassium carbonate (0.39 g, 2.84 mmol), 1,4-dioxane (4.5 mL), and water (0.5 mL), a dichloromethane adduct of dichloro(1,1'-bis(diphenylphosphino)ferrocene)palladium (0.057 g, 0.071 mmol) was added under an argon atmosphere, and the resultant was stirred at 100°C for 6 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.28 g).
ESI-MS: m/z 366.37 [M+1]+.

### [Example 2]

### 2-Fluoro-5-(3-(hydroxymethyl)pyridin-2-yl)-N-((6-methoxypyridin-3-yl)methyl)benzamide

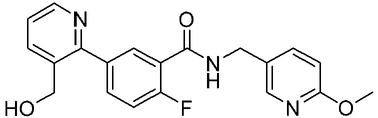

To a mixture of 2-fluoro-5-(3-formylpyridin-2-yl)-N-((6-methoxypyridin-3-yl)methyl)benzamide (0.20 g, 0.54 mmol) and methanol (3.0 mL), sodium borohydride (0.052 g, 1.36 mmol) was added at 0°C, and the resultant was stirred at room temperature for 2 hours. Water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.109 g).
ESI-MS: m/z 368.16 [M+1]+.
1H-NMR (400Hz, DMSO-d6) δ 8.95 (t, J=5.2 Hz, 1H), 8.56 (dd, J=4.8, 1.6 Hz, 1H), 8.13 (d, J=2.0 Hz, 1H), 7.96 (dd, J=8.0, 1.2 Hz, 1H), 7.84-7.82 (m, 1H), 7.79-7.75 (m, 1H), 7.68 (dd, J=8.4, 2.4 Hz, 1H), 7.44-7.36 (m, 2H), 6.80 (d, J = 8.4 Hz, 1H), 5.45 (t, J = 5.6 Hz, 1H), 4.47 (d, J = 5.2 Hz, 2H), 4.40 (d, J = 6.0 Hz, 2H), 3.82 (s, 3H).

### [Production Example 3-1]

### 5-Bromo-3-fluoro-2-methoxypyridine

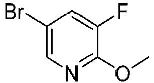

To a mixture of 5-bromo-2,3-difluoropyridine (5.00 g, 25.9 mmol) and methanol (50 mL), sodium methoxide (6.99 g, 129.5 mmol) was added at 0°C, and the resultant was stirred at room temperature for 4 hours. Water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (4.00 g).
1H-NMR (400Hz, CDCl3) δ 7.99 (d, J=2.0 Hz, 1H), 7.47 (dd, J=9.2, 2.0 Hz, 1H), 4.00 (s, 3H).

### [Production Example 3-2]

### 5-Fluoro-6-methoxynicotinonitrile

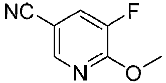

To a mixture of 5-bromo-3-fluoro-2-methoxypyridine (3.0 g, 14.6 mmol), zinc (powder, 0.99 g, 15.3 mmol), zinc cyanide (2.56 g, 21.9 mmol), and DMF (30 mL), tris(dibenzylideneacetone)dipalladium(0) (0.668 g, 0.73 mmol) and 1,1'-bis(diphenylphosphino)ferrocene (0.40 g, 0.73 mmol) were added under an argon atmosphere, and the reaction mixture was stirred at 110°C for 2 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (1.40 g).
1H-NMR (400Hz, CDCl3) δ 8.27 (d, J=2.0 Hz, 1H), 7.53 (dd, J=9.2, 1.6 Hz, 1H), 4.09 (s, 3H).

### [Production Example 3-3]

### tert-Butyl ((5-fluoro-6-methoxypyridin-3-yl)methyl)carbamate

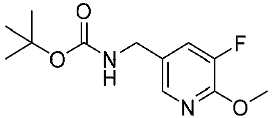

To a mixture of 5-fluoro-6-methoxynicotinonitrile (1.40 g, 9.21 mmol) and methanol (20 mL), nickel(II) chloride (2.97 g, 23.0 mmol) and sodium borohydride (0.87 g, 23.0 mmol) were added at 0°C, the resultant was stirred at the same temperature for 15 minutes, di-tert-butyl dicarbonate (6.05 g, 27.6 mmol) was then added thereto, and the resultant was stirred at 0°C for 2 hours. Ice-cooled water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (1.40 g).
ESI-MS: m/z 257.33 [M+1]+.

### [Production Example 3-4]

### (5-Fluoro-6-methoxypyridin-3-yl)methanamine hydrochloride

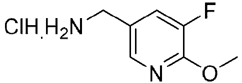

To a mixture of tert-butyl ((5-fluoro-6-methoxypyridin-3-yl)methyl)carbamate (0.85 g, 3.32 mmol) and dichloromethane (10.0 mL), 4 M hydrochloric acid-1,4-dioxane solution (5.0 mL) was added at 0°C, and the resultant was stirred at room temperature for 2 hours. The solvent of the reaction mixture was distilled off under reduced pressure to afford the title compound (0.65 g).
ESI-MS: m/z 157.13 [M+1]+.

### [Production Example 3-5]

### 5-Bromo-2-fluoro-N-((5-fluoro-6-methoxypyridin-3-yl)methyl)benzamide

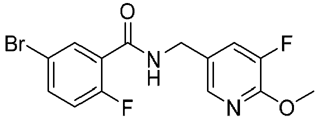

To a mixture of 5-bromo-2-fluorobenzoic acid (0.91 g, 4.18 mmol), (5-fluoro-6-methoxypyridin-3-yl)methanamine hydrochloride (0.80 g, 4.18 mmol), N,N-diisopulethylamine (2.15 g, 16.7 mmol), and dichloromethane (15 mL), 500 propylphosphonic anhydride-ethyl acetate solution (7.97 g, 12.5 mmol) was added, and the reaction mixture was stirred at room temperature for 6 hours. The reaction mixture was diluted with dichloromethane and washed with 1 mol/L hydrochloric acid, water, saturated aqueous solution of sodium hydrogen carbonate, and brine, the organic layer was dried over sodium sulfate, and the solvent was distilled off under reduced pressure. The residue was purified by silica gel column chromatography (hexane/ethyl acetate) to afford the title compound (0.50 g).
ESI-MS: m/z 359.25 [M+3]+.

### [Production Example 3-6]

### 2-Fluoro-N-((5-fluoro-6-methoxypyridin-3-yl)methyl)-5-(4,4,5,5-tetramethyl-1,3,2-dioxaborolan-2-yl)benzamide

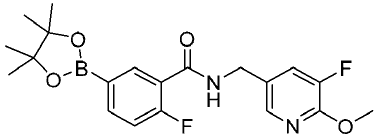

To a mixture of 5-bromo-2-fluoro-N-((5-fluoro-6-methoxypyridin-3-yl)methyl)benzamide (0.50 g, 1.40 mmol), bis(pinacolato)diboron (0.53 g, 2.10 mmol), potassium acetate (0.27 g, 2.80 mmol), and 1,4-dioxane (10 mL), a dichloromethane adduct of dichloro(1,1'-bis(diphenylphosphino)ferrocene)palladium (0.057 g, 0.070 mmol) was added under an argon atmosphere, and the resultant was stirred at 100°C for 6 hours. The temperature of the reaction mixture was returned to room temperature, the reaction mixture was filtered through a Celite, and the solvent of the filtrate was distilled off under reduced pressure. The residue was purified by silica gel column chromatography (hexane/ethyl acetate) to afford the title compound (0.45 g).
ESI-MS: m/z 405.29 [M+1]+.

### [Production Example 3-7]

### 2-Fluoro-N-((5-fluoro-6-methoxypyridin-3-yl)methyl)-5-(3-formylpyridin-2-yl)benzamide

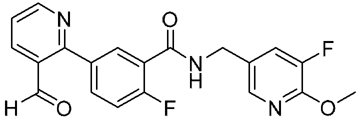

To a mixture of 2-fluoro-N-((5-fluoro-6-methoxypyridin-3-yl)methyl)-5-(4,4,5,5-tetramethyl-1,3,2-dioxaborolan-2-yl)benzamide (0.45 g, 1.11 mmol), 2-bromonicotinaldehyde (0.20 g, 1.11 mmol), potassium carbonate (0.30 g, 2.22 mmol), 1,4-dioxane (4.5 mL), and water (0.5 mL), a dichloromethane adduct of dichloro(1,1'-bis(diphenylphosphino)ferrocene)palladium (0.045 g, 0.050 mmol) was added under an argon atmosphere, and the resultant was stirred at 100°C for 6 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.32 g).
ESI-MS: m/z 382.46 [M-1]-.

### [Example 3]

### 2-Fluoro-N-((5-fluoro-6-methoxypyridin-3-yl)methyl)-5-(3-(hydroxymethyl)pyridin-2-yl)benzamide

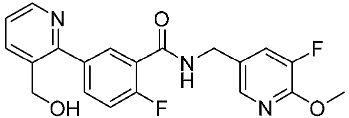

To a mixture of 2-fluoro-N-((5-fluoro-6-methoxypyridin-3-yl)methyl)-5-(3-formylpyridin-2-yl)benzamide (0.32 g, 0.83 mmol) and methanol (4.0 mL), sodium borohydride (0.079 g, 2.08 mmol) was added at 0°C, and the resultant was stirred at room temperature for 2 hours. Water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.109 g).
ESI-MS: m/z 386.15 [M+1]+.
1H NMR (400Hz, DMSO-d6) δ 8.97 (t, J = 5.2 Hz, 1H), 8.56 (d, J = 3.2 Hz, 1H), 7.97-7.95 (m, 2H), 7.86 (dd, J = 6.8, 2.0 Hz, 1H), 7.79-7.76 (m, 1H), 7.63 (dd, J = 11.6, 1.6 Hz, 1H), 7.44-7.37 (m, 2H), 5.44 (t, J = 5.6 Hz, 1H), 4.47 (d, J = 5.2 Hz, 2H), 5.40 (d, J = 6.0Hz, 2H), 3.92 (s, 3H).

### [Production Example 4-1]

### 2-Fluoro-N-((5-fluoro-6-methoxypyridin-3-yl)methyl)-5-(3-(1-hydroxyethyl)pyridin-2-yl)benzamide

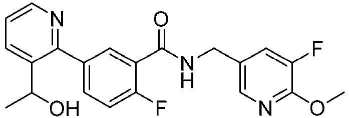

To a mixture of 2-fluoro-N-((5-fluoro-6-methoxypyridin-3-yl)methyl)-5-(3-formylpyridin-2-yl)benzamide (0.50 g, 1.3 mmol) and tetrahydrofuran (6 mL), methylmagnesium bromide (3 M diethyl ether solution, 1.3 mL, 3.9 mmol) was added at 0°C, and the resultant was stirred at room temperature for 15 hours. Saturated aqueous solution of ammonium chloride was added to the reaction mixture, which was subjected to extraction with ethyl acetate. The organic layer was washed with water and brine and dried over sodium sulfate, and the solvent was distilled off under reduced pressure. The residue was purified by silica gel column chromatography (hexane/ethyl acetate) to afford the title compound (230 mg) .

### [Examples 4 and 5]

### (R)-2-Fluoro-N-((5-fluoro-6-methoxypyridin-3-yl)methyl)-5-(3-(1-hydroxyethyl)pyridin-2-yl)benzamide, and (S)-2-fluoro-N-((5-fluoro-6-methoxypyridin-3-yl)methyl)-5-(3-(1-hydroxyethyl)pyridin-2-yl)benzamide

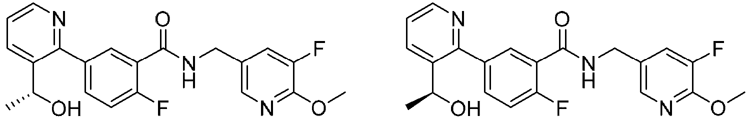

A racemate of 2-fluoro-N-((5-fluoro-6-methoxypyridin-3-yl)methyl)-5-(3-(1-hydroxyethyl)pyridin-2-yl)benzamide (230 mg) was separated into the enantiomers by using supercritical fluid chromatography (SFC) (Lux Cellulose-2 (250 × 21 mm) 5 µm, 0.2% ammonium hydroxide-methanol solution/carbon dioxide). An enantiomer eluted earlier (52 mg, Example 4) and an enantiomer eluted later (62 mg, Example 5) were obtained.

### [Enantiomer eluted earlier]

ESI-MS: m/z 400.18 [M+1]+.

1H-NMR (400 MHz, DMSO-d6) δ 8.98 (bs, 1H), 8.53 (dd, J = 4.8, 1.6 Hz, 1H), 8.03 (dd, J = 8.0, 1.6 Hz, 1H), 7.97 (d, J = 1.6 Hz, 1H), 7.72 (dd, J = 7.2, 2.4 Hz, 1H), 7.67-7.62 (m, 2H), 7.46-7.38 (m, 2H), 5.30 (bs, 1H), 4.78 (q, J = 6.4 Hz, 1H), 4.43 (s, 2H), 3.92 (s, 3H), 1.27 (d, J = 6.4 Hz, 3H).

### [Enantiomer eluted later]

ESI-MS: m/z 400.18 [M+1]+.

1H-NMR (400 MHz, DMSO-d6) δ 8.98 (t, J = 5.6 Hz, 1H), 8.53 (dd, J = 4.8, 1.6 Hz, 1H), 8.03 (dd, J = 8.0, 1.6 Hz, 1H), 7.97 (d, J = 1.6 Hz, 1H), 7.73 (dd, J = 7.2, 2.4 Hz, 1H), 7.67-7.62 (m, 2H), 7.46-7.38 (m, 2H), 5.29 (bs, 1H), 4.79 (q, J = 6.4 Hz, 1H), 4.43 (s, 2H), 3.92 (s, 3H), 1.27 (d, J = 6.4 Hz, 3H).

### [Production Example 6-1]

### 5-Bromo-2-(difluoromethoxy)pyridine

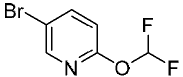

To a mixture of 5-bromopyridin-2-ol (5.0 g, 28.7 mmol) and N,N-dimethylformamide (10 mL), 60% sodium hydride (1.72 g, 43.1 mmol) was slowly added at 0°C, and the resultant was stirred at room temperature for 30 minutes. To the reaction mixture, 2,2-difluoro-2-(fluorosulfonyl)acetic acid (7.67 g, 43.1 mmol) was slowly added, and the resultant was stirred at room temperature for 4 hours. Ice-cooled water was added to the reaction mixture, which was washed with ethyl acetate. To the aqueous layer, 2 N hydrochloric acid was added, and the aqueous layer was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (2.4 g).
1H-NMR (400 MHz, DMSO-d6) δ 8.42 (d, J = 2.4 Hz, 1H), 8.15 (dd, J = 8.4, 2.4 Hz, 1H), 7.6 (t, J = 72.4 Hz, 1H), 7.11 (d, J = 8.4 Hz, 1H).

### [Production Example 6-2]

### 6-(Difluoromethoxy)nicotinonitrile

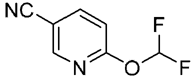

To a mixture of 5-bromo-2-(difluoromethoxy)pyridine (2.4 g, 10.7 mmol), zinc (powder, 0.70 g, 10.7 mmol), zinc cyanide (1.88 g, 16.1 mmol), and DMF (25 mL), tris(dibenzylideneacetone)dipalladium(0) (0.48 g, 0.53 mmol) and 1,1'-bis(diphenylphosphino)ferrocene (0.29 g, 0.53 mmol) were added under an argon atmosphere, and the reaction mixture was stirred at 100°C for 2 hours. The temperature of the reaction mixture was returned to room temperature, ice-cooled water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (1.0 g).
1H-NMR (400 MHz, CDCl3) δ 8.52 (d, J = 2.4 Hz, 1H), 7.99 (dd, J = 8.4, 2.4 Hz, 1H), 7.5 (t, J = 71.6 Hz, 1H), 7.11 (d, J = 8.4 Hz, 1H).

### [Production Example 6-3]

### tert-Butyl((6-(difluoromethoxy)pyridin-3-yl)methyl)carbamate

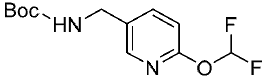

To a mixture of 6-(difluoromethoxy)nicotinonitrile (1.0 g, 5.88 mmol) and methanol (10 mL), nickel(II) chloride (1.90 g, 14.7 mmol) and sodium borohydride (0.44 g, 11.8 mmol) were added at 0°C, the resultant was stirred at the same temperature for 15 minutes, di-tert-butyl dicarbonate (3.20 g, 14.7 mmol) was then added thereto, and the resultant was stirred at room temperature for 2 hours. Ice-cooled water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.76 g).
ESI-MS: m/z 275.18 [M+1]+.

### [Production Example 6-4]

### (6-(Difluoromethoxy)pyridin-3-yl)methanamine hydrochloride

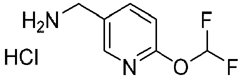

To a mixture of tert-butyl ((6-(difluoromethoxy)pyridin-3-yl)methyl)carbamate (0.75 g, 2.73 mmol) and dichloromethane (10 mL), 4 M hydrochloric acid-1,4-dioxane solution (5 mL) was added at 0°C, and the resultant was stirred at room temperature for 2 hours. The solvent of the reaction mixture was distilled off under reduced pressure to afford the title compound (0.60 g).
ESI-MS: m/z 175.16 [M+1]+.

### [Production Example 6-5]

### 5-Bromo-N-((6-(difluoromethoxy)pyridin-3-yl)methyl)-2-fluorobenzamide

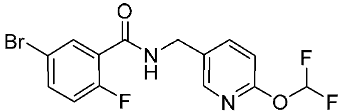

To a mixture of 5-bromo-2-fluorobenzoic acid (0.62 g, 2.84 mmol), (6-(difluoromethoxy)pyridin-3-yl)methanamine hydrochloride (0.60 g, 2.84 mmol), N,N-diisopulethylamine (1.47 g, 11.4 mmol), and dichloromethane (10 mL), 500 propylphosphonic anhydride-ethyl acetate solution (2.71 g, 8.52 mmol) was added, and the reaction mixture was stirred at room temperature for 6 hours. The reaction mixture was diluted with dichloromethane and washed with 1 mol/L hydrochloric acid, water, saturated aqueous solution of sodium hydrogen carbonate, and brine, the organic layer was dried over sodium sulfate, and the solvent was distilled off under reduced pressure. The residue was purified by silica gel column chromatography (hexane/ethyl acetate) to afford the title compound (0.50 g).
ESI-MS: m/z 374.97 [M+1]+.

### [Production Example 6-6]

### N-((6-(Difluoromethoxy)pyridin-3-yl)methyl)-2-fluoro-5-(4,4,5,5-tetramethyl-1,3,2-dioxaborolan-2-yl)benzamide

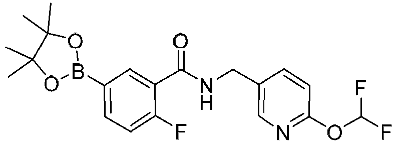

To a mixture of 5-bromo-N-((6-(difluoromethoxy)pyridin-3-yl)methyl)-2-fluorobenzamide (0.50 g, 1.33 mmol), bis(pinacolato)diboron (0.50 g, 2.00 mmol), potassium acetate (0.26 g, 2.66 mmol), and 1,4-dioxane (5 mL), a dichloromethane adduct of dichloro(1,1'-bis(diphenylphosphino)ferrocene)palladium (0.055 g, 0.066 mmol) was added under an argon atmosphere, and the resultant was stirred at 100°C for 6 hours. The temperature of the reaction mixture was returned to room temperature, the reaction mixture was filtered through a Celite, and the solvent of the filtrate was distilled off under reduced pressure. The residue was purified by silica gel column chromatography (hexane/ethyl acetate) to afford the title compound (0.45 g).
ESI-MS: m/z 423.10 [M+1]+.

### [Production Example 6-7]

### N-((6-(Difluoromethoxy)pyridin-3-yl)methyl)-2-fluoro-5-(3-formylpyridin-2-yl)benzamide

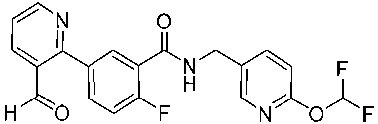

To a mixture of N-((6-(difluoromethoxy)pyridin-3-yl)methyl)-2-fluoro-5-(4,4,5,5-tetramethyl-1,3,2-dioxaborolan-2-yl)benzamide (0.45 g, 1.06 mmol), 2-bromonicotinaldehyde (0.20 g, 1.06 mmol), potassium carbonate (0.29 g, 2.12 mmol), 1,4-dioxane (4.5 mL), and water (0.5 mL), a dichloromethane adduct of dichloro(1,1'-bis(diphenylphosphino)ferrocene)palladium (0.04 g, 0.05 mmol) was added under an argon atmosphere, and the resultant was stirred at 100°C for 6 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.22 g).
ESI-MS: m/z 402.3 [M+1]+.

### [Example 6]

### N-((6-(Difluoromethoxy)pyridin-3-yl)methyl)-2-fluoro-5-(3-(hydroxymethyl)pyridin-2-yl)benzamide

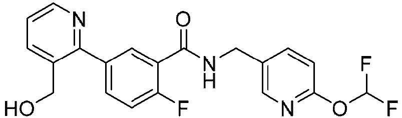

To a mixture of N-((6-(difluoromethoxy)pyridin-3-yl)methyl)-2-fluoro-5-(3-formylpyridin-2-yl)benzamide (0.3 g, 0.75 mmol) and methanol (5 mL), sodium borohydride (0.04 g, 1.1 mmol) was added at 0°C, and the resultant was stirred at room temperature for 3 hours. Water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.065 g).
ESI-MS: m/z 404.15 [M+1]+.
1H- (400 MHz, DMSO-d6) δ 9.02 (t, J = 5.4 Hz, 1H), 8.56 (d, J = 7.6 Hz, 1H), 8.23 (d, J = 2.0 Hz, 1H), 7.96 (d, J = 8.0 Hz, 1H), 7.90-7.51 (m, 4 H), 7.44-7.37 (m, 2H), 7.08 (d, J = 8.4 Hz, 1H), 5.44 (t, J = 5.0 Hz, 1H), 4.48-4.73 (m, 4H).

### [Production Example 7-1]

### 5-Bromo-2-ethoxy-3-fluoropyridine

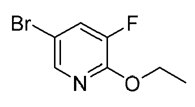

To a mixture of 5-bromo-2,3-difluoropyridine (7.2 g, 37.2 mmol) and dimethyl sulfoxide (120 mL), cesium carbonate (36.3 g, 111 mmol) and ethanol (8 mL, 137 mmol) were added, and the resultant was stirred at 80°C for 6 hours. The temperature of the reaction mixture was returned to room temperature, and the reaction mixture was filtered through a Celite while being washed with ether. The organic layer was washed with brine and dried over sodium sulfate, and the solvent was distilled off under reduced pressure. The residue was purified by silica gel column chromatography (hexane/ethyl acetate) to afford the title compound (3.5 g).
1H NMR (400 MHz, DMSO-d6) δ 8.12 (s, 1H), 8.07 (d, J = 11.2 Hz, 1H), 4.37 (q, J = 7.2 Hz, 2H), 1.34 (t, J = 7.2 Hz, 3H).

### [Production Example 7-2]

### 6-Ethoxy-5-fluoronicotinonitrile

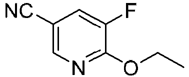

To a mixture of 5-bromo-2-ethoxy-3-fluoropyridine (3.0 g, 13.6 mmol), zinc (powder, 0.93 g, 14.3 mmol), zinc cyanide (1.60 g, 13.6 mmol), and DMF (25 mL), tris(dibenzylideneacetone)dipalladium(0) (0.62 g, 0.68 mmol) and 1,1'-bis(diphenylphosphino)ferrocene (0.38 g, 0.68 mmol) were added under an argon atmosphere, and the reaction mixture was stirred at 100°C for 2 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (1.20 g).
1H NMR (400 MHz, DMSO-d6) δ 8.52 (d, J = 2.0 Hz, 1H), 8.27 (dd, J = 12.4 Hz, 2.0 Hz, 1H), 4.46 (q, J = 7.2 Hz, 2H), 1.36 (t, J = 7.2 Hz, 3H).

### [Production Example 7-3]

### tert-Butyl ((6-ethoxy-5-fluoropyridin-3-yl)methyl)carbamate

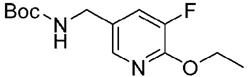

To a mixture of 6-ethoxy-5-fluoronicotinonitrile (1.20 g, 7.22 mmol) and methanol (20 mL), nickel(II) chloride (1.40 g, 10.8 mmol) and sodium borohydride (0.68 g, 18.1 mmol) were added at 0°C, the resultant was stirred at the same temperature for 15 minutes, di-tert-butyl dicarbonate (6.30 g, 28.9 mmol) was then added thereto, and the resultant was stirred at 0°C for 2 hours. Ice-cooled water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.80 g).
ESI-MS: m/z 271.13 [M+1]+.

### [Production Example 7-4]

### (6-Ethoxy-5-fluoropyridin-3-yl)methanamine hydrochloride

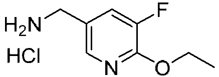

To a mixture of tert-butyl ((6-ethoxy-5-fluoropyridin-3-yl)methyl)carbamate (0.85 g, 3.14 mmol) and dichloromethane (10.0 mL), 4 M hydrochloric acid-1,4-dioxane solution (5.0 mL) was added at 0°C, and the resultant was stirred at room temperature for 2 hours. The solvent of the reaction mixture was distilled off under reduced pressure, and the residue was washed with ether to afford the title compound (0.60 g).
ESI-MS: m/z 171.15 [M+1]+.

### [Production Example 7-5]

### 5-Bromo-N-((6-ethoxy-5-fluoropyridin-3-yl)methyl)-2-fluorobenzamide

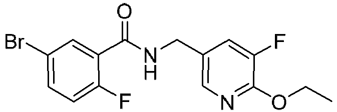

To a mixture of 5-bromo-2-fluorobenzoic acid (0.58 g, 2.67 mmol), (6-ethoxy-5-fluoropyridin-3-yl)methanamine hydrochloride (0.55 g, 2.67 mmol), N,N-diisopulethylamine (1.38 g, 10.7 mmol), and dichloromethane (15 mL), 500 propylphosphonic anhydride-ethyl acetate solution (5.1 g, 8.0 mmol) was added, and the reaction mixture was stirred at room temperature for 5 hours. The reaction mixture was diluted with dichloromethane and washed with 1 mol/L hydrochloric acid, water, saturated aqueous solution of sodium hydrogen carbonate, and brine, the organic layer was dried over sodium sulfate, and the solvent was distilled off under reduced pressure. The residue was purified by silica gel column chromatography (hexane/ethyl acetate) to afford the title compound (0.40 g).
ESI-MS: m/z 370.99 [M+1]+.

### [Production Example 7-6]

### N-((6-Ethoxy-5-fluoropyridin-3-yl)methyl)-2-fluoro-5-(4,4,5,5-tetramethyl-1,3,2-dioxaborolan-2-yl)benzamide

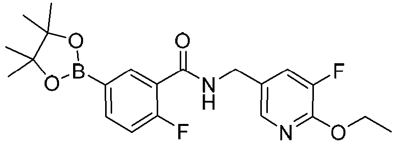

To a mixture of 5-bromo-N-((6-ethoxy-5-fluoropyridin-3-yl)methyl)-2-fluorobenzamide (0.40 g, 1.08 mmol), bis(pinacolato)diboron (0.41 g, 1.62 mmol), potassium acetate (0.212 g, 2.16 mmol), and 1,4-dioxane (10 mL), a dichloromethane adduct of dichloro(1,1'-bis(diphenylphosphino)ferrocene)palladium (0.044 g, 0.050 mmol) was added under an argon atmosphere, and the resultant was stirred at 100°C for 6 hours. The temperature of the reaction mixture was returned to room temperature, the reaction mixture was filtered through a Celite, and the solvent of the filtrate was distilled off under reduced pressure. The residue was purified by silica gel column chromatography (hexane/ethyl acetate) to afford the title compound (0.30 g).
ESI-MS: m/z 419.13 [M+1]+.

### [Example 7]

### N-((6-Ethoxy-5-fluoropyridin-3-yl)methyl)-2-fluoro-5-(3-(hydroxymethyl)pyridin-2-yl)benzamide

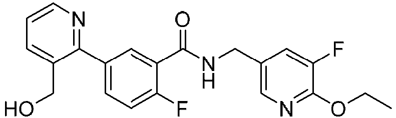

To a mixture of N-((6-ethoxy-5-fluoropyridin-3-yl)methyl)-2-fluoro-5-(4,4,5,5-tetramethyl-1,3,2-dioxaborolan-2-yl)benzamide (0.25 g, 0.59 mmol), (2-bromopyridin-3-yl)methanol (0.11 g, 0.59 mmol), potassium carbonate (0.16 g, 1.18 mmol), 1,4-dioxane (4.5 mL), and water (0.5 mL), a dichloromethane adduct of dichloro(1,1'-bis(diphenylphosphino)ferrocene)palladium (0.024 g, 0.030 mmol) was added under an argon atmosphere, and the resultant was stirred at 100°C for 6 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.064 g).
ESI-MS: m/z 400.4 [M+1]+.
1H NMR (400 MHz, DMSO-d6) δ 8.96 (t, J = 5.6 Hz, 1H), 8.56 (d, J = 4.8 Hz, 1H), 7.97-7.95 (m, 2H), 7.86 (dd, J = 7.2, 2.4 Hz, 1H), 7.79-7.76 (m, 1H), 7.62 (dd, J = 11.6, 2.0 Hz, 1H), 7.45-7.37 (m, 2H), 5.43 (t, J = 5.6 Hz, 1H), 4.48 (d, J = 5.2 Hz, 2H), 4.43 (d, J = 5.6 Hz, 2H), 4.37 (q, J = 7.2 Hz, 2H), 3.92 (t, J = 7.2 Hz, 3H).

### [Production Example 8-1]

### 5-Bromo-3-fluoro-2-isopropoxypyridine

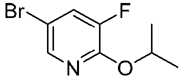

To a mixture of 5-bromo-2,3-difluoropyridine (5.0 g, 25.9 mmol) and dimethyl sulfoxide (75 mL), cesium carbonate (25.1 g, 77.7 mmol) and 2-propanol (5.9 mL, 77.7 mmol) were added, and the resultant was stirred at 80°C for 6 hours. The temperature of the reaction mixture was returned to room temperature, and the reaction mixture was filtered through a Celite while being washed with ether. The organic layer was washed with brine and dried over sodium sulfate, and the solvent was distilled off under reduced pressure. The residue was purified by silica gel column chromatography (hexane/ethyl acetate) to afford the title compound (3.6 g).
1H NMR (400 MHz, DMSO-d6) δ 8.11 (d, J = 2.0 Hz, 1H), 8.06 (d, J = 10.0 Hz, 2 Hz, 1H), 5.26 (m, 1H), 1.30 (d, J = 6.0 Hz, 6H).

### [Production Example 8-2]

### 5-Fluoro-6-isopropoxynicotinonitrile

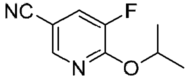

To a mixture of 5-bromo-3-fluoro-2-isopropoxypyridine (3.6 g, 15.4 mmol), zinc (powder, 1.11 g, 16.9 mmol), zinc cyanide (2.72 g, 23.1 mmol), and DMF (45 mL), tris(dibenzylideneacetone)dipalladium(0) (0.70 g, 0.77 mmol) and 1,1'-bis(diphenylphosphino)ferrocene (0.42 g, 0.77 mmol) were added under an argon atmosphere, and the reaction mixture was stirred at 100°C for 6 hours. The temperature of the reaction mixture was returned to room temperature, the reaction mixture was filtered through a Celite, and the solvent of the filtrate was distilled off under reduced pressure. The residue was purified by silica gel column chromatography (hexane/ethyl acetate) to afford the title compound (1.90 g).
1H NMR (400 MHz, DMSO-d6) δ 8.50 (dd, J = 7.8 Hz, 2.0 Hz, 1H), 8.26 (dd, J = 10.8 Hz, 1.6 Hz, 1H), 5.38 (m, 1H), 1.34 (d, J = 6.4 Hz, 6H).

### [Production Example 8-3]

### tert-Butyl ((5-fluoro-6-isopropoxypyridin-3-yl)methyl)carbamate

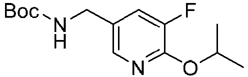

To a mixture of 5-fluoro-6-isopropoxynicotinonitrile (1.90 g, 10.6 mmol) and methanol (30 mL), nickel(II) chloride (1.36 g, 15.8 mmol) and sodium borohydride (0.99 g, 26.3 mmol) were added at 0°C, the resultant was stirred at the same temperature for 15 minutes, di-tert-butyl dicarbonate (2.30 g, 42.2 mmol) was then added thereto, and the resultant was stirred at 0°C for 2 hours. Ice-cooled water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.95 g).
ESI-MS: m/z 285.14 [M+1]+.

### [Production Example 8-4]

### (5-Fluoro-6-isopropoxypyridin-3-yl)methanamine hydrochloride

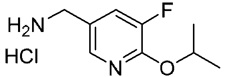

To a mixture of tert-butyl ((5-fluoro-6-isopropoxypyridin-3-yl)methyl)carbamate (1.10 g, 3.87 mmol) and dichloromethane (10 mL), 4 M hydrochloric acid-1,4-dioxane solution (8 mL) was added at 0°C, and the resultant was stirred at room temperature for 6 hours. The solvent of the reaction mixture was distilled off under reduced pressure, and the residue was washed with ether to afford the title compound (0.80 g).
ESI-MS: m/z 185.16 [M+1]+.

### [Production Example 8-5]

### 5-Bromo-2-fluoro-N-((5-fluoro-6-isopropoxypyridin-3-yl)methyl)benzamide

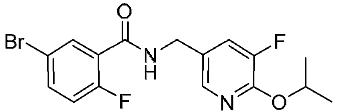

To a mixture of 5-bromo-2-fluorobenzoic acid (0.69 g, 3.18 mmol), (5-fluoro-6-isopropoxypyridin-3-yl)methanamine hydrochloride (0.70 g, 3.18 mmol), N,N-diisopulethylamine (1.64 g, 12.7 mmol), and dichloromethane (10 mL), 50% propylphosphonic anhydride-ethyl acetate solution (3.63 g, 9.54 mmol) was added, and the reaction mixture was stirred at room temperature for 16 hours. Water was added to the reaction mixture, which was subjected to extraction with ethyl acetate. The organic layer was washed with saturated aqueous solution of sodium hydrogen carbonate and brine and dried over sodium sulfate, and the solvent was distilled off under reduced pressure. The residue was pulverized and washed with pentane to afford the title compound (0.40 g). ESI-MS: m/z 385.02 [M+1]+.

### [Production Example 8-6]

### 2-Fluoro-N-((5-fluoro-6-isopropoxypyridin-3-yl)methyl)-5-(4,4,5,5-tetramethyl-1,3,2-dioxaborolan-2-yl)benzamide

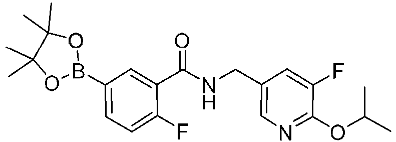

To a mixture of 5-bromo-2-fluoro-N-((5-fluoro-6-isopropoxypyridin-3-yl)methyl)benzamide (0.45 g, 1.17 mmol), bis(pinacolato)diboron (0.44 g, 1.75 mmol), potassium acetate (0.230 g, 2.34 mmol), and 1,4-dioxane (8 mL), a dichloromethane adduct of dichloro(1,1'-bis(diphenylphosphino)ferrocene)palladium (0.048 g, 0.05 mmol) was added under an argon atmosphere, and the resultant was stirred at 100°C for 16 hours. The temperature of the reaction mixture was returned to room temperature, the reaction mixture was filtered through a Celite, and the solvent of the filtrate was distilled off under reduced pressure. The residue was purified by silica gel column chromatography (hexane/ethyl acetate) to afford the title compound (0.30 g).
ESI-MS: m/z 433.20 [M+1]+.

### [Example 8]

### 2-Fluoro-N-((5-fluoro-6-isopropoxypyridin-3-yl)methyl)-5-(3-(hydroxymethyl)pyridin-2-yl)benzamide

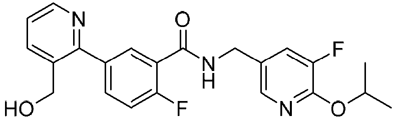

To a mixture of 2-fluoro-N-((5-fluoro-6-isopropoxypyridin-3-yl)methyl)-5-(4,4,5,5-tetramethyl-1,3,2-dioxaborolan-2-yl)benzamide (0.25 g, 0.58 mmol), (2-bromopyridin-3-yl)methanol (0.11 g, 0.58 mmol), potassium carbonate (0.16 g, 1.16 mmol), 1,4-dioxane (3.6 mL), and water (0.4 mL), a dichloromethane adduct of dichloro(1,1'-bis(diphenylphosphino)ferrocene)palladium (0.024 g, 0.03 mmol) was added under an argon atmosphere, and the resultant was stirred at 100°C for 6 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.04 g).
ESI-MS: m/z 414.4 [M+1]+.
1H NMR (400 MHz, DMSO-d6) δ 8.95 (t, J = 5.6 Hz, 1H), 8.56 (dd, J = 4.8, 1.6 Hz, 1H), 7.97-7.94 (m, 2H), 7.85 (dd, J = 6.8, 2.4 Hz, 1H), 7.79-7.75 (m, 1H), 7.60 (dd, J = 11.2, 1.6 Hz, 1H), 7.44-7.37 (m, 2H), 5.43 (t, J = 5.2 Hz, 1H), 5.32-5.26 (m, 1H), 4.48 (d, J = 5.2 Hz, 2H), 4.42 (d, J = 5.6 Hz, 2H), 1.31 (d, J = 6.4 Hz, 6H).

### [Production Example 9-1]

### 5-Bromo-2-cyclopropoxy-3-fluoropyridine

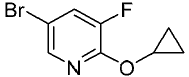

To a mixture of 5-bromo-2,3-difluoropyridine (6.0 g, 30.9 mmol) and dimethyl sulfoxide (70 mL), cesium carbonate (30.2 g, 92.8 mmol) and cyclopropanol (5.38 g, 92.8 mmol) were added, and the resultant was stirred at 80°C for 2 hours. The temperature of the reaction mixture was returned to room temperature, and the reaction mixture was filtered through a Celite while being washed with ether. The organic layer was washed with brine and dried over sodium sulfate, and the solvent was distilled off under reduced pressure. The residue was purified by silica gel column chromatography (hexane/ethyl acetate) to afford the title compound (3.2 g).
1H-NMR (400 MHz, DMSO-d6) δ 8.18 (d, J = 2.0 Hz, 1H), 8.08 (dd, J = 10.0, 2.4 Hz, 1H), 4.32-4.28 (m, 1H), 0.84-0.72 (m, 4H).

### [Production Example 9-2]

### 6-Cyclopropoxy-5-fluoronicotinonitrile

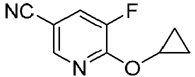

To a mixture of 5-bromo-2-cyclopropoxy-3-fluoropyridine (3.2 g, 13.8 mmol), zinc (powder, 0.95 g, 14.5 mmol), zinc cyanide (1.62 g, 13.8 mmol), and DMF (30 mL), tris(dibenzylideneacetone)dipalladium(0) (0.64 g, 0.70 mmol) and 1,1'-bis(diphenylphosphino)ferrocene (0.46 g, 0.70 mmol) were added under an argon atmosphere, and the reaction mixture was stirred at 100°C for 2 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (1.70 g).
1H-NMR (400 MHz, DMSO-d6) δ 8.58 (d, J = 2.0 Hz, 1H), 8.30 (dd, J = 10.4, 2.0 Hz, 1H), 4.32-4.28 (m, 1H), 0.84-0.72 (m, 4H).

### [Production Example 9-3]

### tert-Butyl ((6-cyclopropoxy-5-fluoropyridin-3-yl)methyl)carbamate

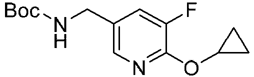

To a mixture of 6-cyclopropoxy-5-fluoronicotinonitrile (1.50 g, 8.42 mmol) and methanol (20 mL), nickel(II) chloride (1.63 g, 12.6 mmol) and sodium borohydride (0.80 g, 21.0 mmol) were added at 0°C, the resultant was stirred at the same temperature for 15 minutes, di-tert-butyl dicarbonate (7.35 g, 33.7 mmol) was then added thereto, and the resultant was stirred at room temperature for 2 hours. Ice-cooled water was added to the reaction mixture, methanol in the reaction mixture was distilled off under reduced pressure, and the residue was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.90 g).
1H-NMR (400 MHz, DMSO-d6) δ 7.81 (d, J = 1.2 Hz, 1H), 7.46 (dd, J = 11.2, 2.0 Hz, 1H), 5.32-4.23 (m, 1H), 4.06 (d, J = 6.0 Hz, 2H), 1.34 (s, 9H), 1.29-1.15 (m, 4H).

### [Production Example 9-4]

### (6-Cyclopropoxy-5-fluoropyridin-3-yl)methanamine hydrochloride

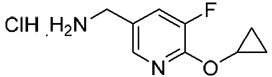

To a mixture of tert-butyl ((6-cyclopropoxy-5-fluoropyridin-3-yl)methyl)carbamate (0.90 g, 3.18 mmol) and dichloromethane (10.0 mL), 4 M hydrochloric acid-1,4-dioxane solution (5.0 mL) was added at 0°C, and the resultant was stirred at room temperature for 2 hours. The solvent of the reaction mixture was distilled off under reduced pressure, and the residue was washed with ether to afford the title compound (0.70 g).
1H-NMR (400 MHz, DMSO) δ 8.55 (bs, 1H), 8.17 (d, J = 1.6 Hz, 1H), 7.90 (dd, J = 11.6, 2.0 Hz, 1H), 5.91 (bs, 1H), 4.34(m, 1H), 4.01 (dd, J = 11.2, 5.6 Hz, 1H), 0.81-0.76 (m, 4H).

### [Production Example 9-5]

### 5-Bromo-N-((6-cyclopropoxy-5-fluoropyridin-3-yl)methyl)-2-fluorobenzamide

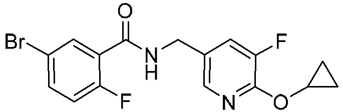

To a mixture of 5-bromo-2-fluorobenzoic acid (0.65 g, 2.97 mmol), (6-cyclopropoxy-5-fluoropyridin-3-yl)methanamine hydrochloride (0.65 g, 2.97 mmol), N,N-diisopulethylamine (1.15 g, 8.94 mmol), and dichloromethane (10 mL), 50% propylphosphonic anhydride-ethyl acetate solution (3.77 g, 11.9 mmol) was added, and the reaction mixture was stirred at room temperature for 6 hours. The reaction mixture was diluted with dichloromethane and washed with 1 mol/L hydrochloric acid, water, saturated aqueous solution of sodium hydrogen carbonate, and brine, the organic layer was dried over sodium sulfate, and the solvent was distilled off under reduced pressure. The residue was purified by silica gel column chromatography (hexane/ethyl acetate) to afford the title compound (0.37 g).
ESI-MS: m/z 383.13 [M+1]+.

### [Production Example 9-6]

### N-((6-Cyclopropoxy-5-fluoropyridin-3-yl)methyl)-2-fluoro-5-(4,4,5,5-tetramethyl-1,3,2-dioxaborolan-2-yl)benzamide

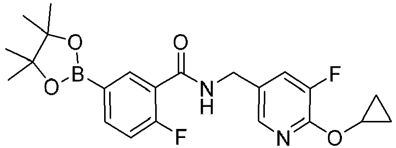

To a mixture of 5-bromo-N-((6-cyclopropoxy-5-fluoropyridin-3-yl)methyl)-2-fluorobenzamide (0.37 g, 0.97 mmol), bis(pinacolato)diboron (0.37 g, 1.45 mmol), potassium acetate (0.19 g, 1.94 mmol), and 1,4-dioxane (5 mL), a dichloromethane adduct of dichloro(1,1'-bis(diphenylphosphino)ferrocene)palladium (0.04 g, 0.05 mmol) was added under an argon atmosphere, and the resultant was stirred at 100°C for 6 hours. The temperature of the reaction mixture was returned to room temperature, the reaction mixture was filtered through a Celite, and the solvent of the filtrate was distilled off under reduced pressure. The residue was purified by silica gel column chromatography (hexane/ethyl acetate) to afford the title compound (0.27 g).
ESI-MS: m/z 431.34 [M+1]+.

### [Example 9]

### N-((6-Cyclopropoxy-5-fluoropyridin-3-yl)methyl)-2-fluoro-5-(3-(hydroxymethyl)pyridin-2-yl)benzamide

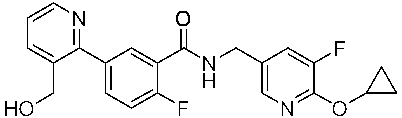

To a mixture of N-((6-cyclopropoxy-5-fluoropyridin-3-yl)methyl)-2-fluoro-5-(4,4,5,5-tetramethyl-1,3,2-dioxaborolan-2-yl)benzamide (0.22 g, 0.51 mmol), (2-bromopyridin-3-yl)methanol (0.095 g, 0.51 mmol), potassium carbonate (0.14 g, 1.02 mmol), 1,4-dioxane (4.5 mL), and water (0.5 mL), a dichloromethane adduct of dichloro(1,1'-bis(diphenylphosphino)ferrocene)palladium (0.021 g, 0.025 mmol) was added under an argon atmosphere, and the resultant was stirred at 100°C for 6 hours. The temperature of the reaction mixture was returned to room temperature, water was added thereto, and the resultant was subjected to extraction with ethyl acetate. The organic layer was washed with brine and dried over sodium sulfate, and the solvent was distilled off under reduced pressure. The residue was purified by silica gel column chromatography (hexane/ethyl acetate) to afford the title compound (0.083 g).
ESI-MS: m/z 412.3 [M+1]+.
1H-NMR (400 MHz, DMSO-d6) δ 8.97 (t, J = 5.0 Hz, 1H), 8.55 (d, J = 4.0 Hz, 1H), 8.05 (br s, 1H), 9.96 (d, J = 7.6 Hz, 1H), 7.85 (dd, J = 6.8, 2.0 Hz, 1H), 7.79-7.78 (m, 1H), 7.62 (d, J = 11.2 Hz, 1H), 7.44-7.37 (m, 2H), 5.44 (t, J = 5.2 Hz, 1H), 4.48 (d, J = 5.2 Hz, 2H), 4.44 (d, J = 6.0 Hz, 2H), 4.34-4.30 (m, 1H), 0.80-0.65 (m, 4H).

### [Comparative Example 1]

A compound below, which is a commercially available product, was used as Comparative Example 1. The following compound is a compound described in International Publication No. WO 2008/112164.

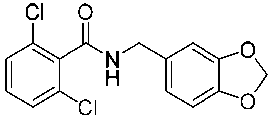

### [Comparative Example 2]

The following compound was synthesized in accordance with a method described in International Publication No. WO 2015/127137, and used as Comparative Example 2.

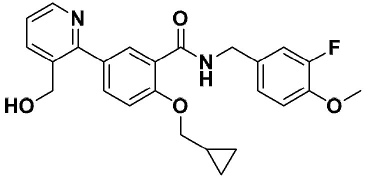

### [Comparative Example 3]

The following compound was synthesized in accordance with a method described in International Publication No. WO 2019/151241, and used as Comparative Example 3.

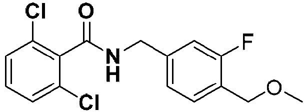

### [Comparative Example 4]

The following compound was synthesized in accordance with a method described in International Publication No. WO 2019/151241, and used as Comparative Example 4.

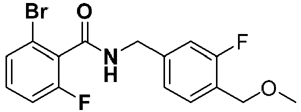

### [Comparative Example 5]

The following compound was synthesized in accordance with a method described in International Publication No. WO 2019/151241, and used as Comparative Example 5.

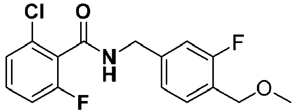

### [Comparative Example 6]

The following compound was synthesized in accordance with a method described in International Publication No. WO 2019/151241, and used as Comparative Example 6.

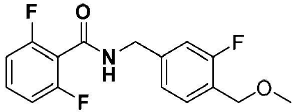

### [Test Example 1-1: ALDH2 Activation Effect]

The activation effect of each test compound on the acetaldehyde oxidation rate of ALDH2 was measured with the following method. A test was conducted by using a commercially available PicoProbe (TM) Aldehyde dehydrogenase Activity Assay Kit (manufactured by BioVision Inc.) with reference to an instruction attached to the product under the following conditions. To each well of a 384-well plate, the following (1) to (3) were sequentially added with a pipette:
(1) 10 µL of solution obtained by dissolving and diluting a human recombinant ALDH2 enzyme (manufactured by Abcam, product number: ab87415) with ALDH assay Buffer (final concentration: 2 µg/mL);
(2) 10 µL of 30 µM or 90 µM test compound solution (with 3% DMSO) prepared by using ALDH assay Buffer; and
(3) 10 µL of Reaction mix composed with ratio of 8.6 µL of ALDH assay Buffer, 0.6 µL of PicoProbe, 0.3 µL of Substrate Mix, and 1.5 µL of Acetaldehyde.

Before addition of (3), incubation was performed at room temperature for 5 minutes. After addition of (3), fluorescence intensity was measured at room temperature every 2.5 minutes over a period of 180 minutes by using an EnSight (manufactured by PerkinElmer Inc.) to determine the oxidation rate. Wavelengths of 535 nm and 587 nm were used for fluorescence intensities of excitation and emission, respectively.

Table 1 shows acetaldehyde oxidation rates with addition of different test compounds as the acetaldehyde oxidation rate with addition of no test compound was assumed as 100%. The results in Table 1 confirmed that the compounds of Examples 1 to 3 each have ALDH2 activation effect.

### [Table 1]

**Table 1**

| | Concentration of test compound | |
|---|---|---|
| | 10 µM | 30 µM |
| Example 1 | 123 | 130 |
| Example 2 | 150 | 180 |
| Example 3 | 188 | 226 |
| Comparative Example 1 | 199 | 225 |

### [Test Example 1-2: ALDH2 Activation Effect]

The activation effect of each test compound on the acetaldehyde oxidation rate of ALDH2 was measured with the following method. A test was conducted by using a commercially available PicoProbe (TM) Aldehyde dehydrogenase Activity Assay Kit (manufactured by Abcam) with reference to an instruction attached to the product under the following conditions. To each well of a 384-well plate, the following (1) to (3) were sequentially added with a pipette:
(1) 10 µL of solution obtained by dissolving and diluting a human recombinant ALDH2 enzyme (manufactured by Abcam, product number: ab87415) with ALDH assay Buffer (final concentration: 1 µg/mL);
(2) 10 µL of 30 µM or 90 µM test compound solution (with 3% DMSO) prepared by using ALDH assay Buffer; and
(3) 10 µL of Reaction mix composed with ratio of 7.3 µL of ALDH assay Buffer, 0.6 µL of PicoProbe, 0.6 µL of Substrate Mix, and 1.5 µL of Acetaldehyde.

Before addition of (3), incubation was performed at room temperature for 5 minutes. After addition of (3), fluorescence intensity was measured at room temperature every 2.5 minutes over a period of 60 minutes by using an Envision (manufactured by PerkinElmer Inc.) to determine the oxidation rate. Wavelengths of 535 nm and 587 nm were used for fluorescence intensities of excitation and emission, respectively.

Table 2 shows acetaldehyde oxidation rates with addition of different test compounds as the acetaldehyde oxidation rate with addition of no test compound was assumed as 100%. The results in Table 2 confirmed that the compounds of Examples 4 to 9 each have ALDH2 activation effect.

### [Table 2]

**Table 2**

| | Concentration of test compound | |
|---|---|---|
| | 10 µM | 30 µM |
| Example 4 | 133 | 155 |
| Example 5 | 122 | 170 |
| Example 6 | 125 | 139 |
| Example 7 | 331 | 415 |
| Example 8 | 154 | 224 |
| Example 9 | 182 | 283 |
| Comparative Example 1 | 220 | 275 |

### [Test Example 2: Metabolic Stability with Mouse Hepatic Microsomes]

Metabolic stability in mouse hepatic microsomes was evaluated with the following method.

### 1. Materials

(1) Mouse hepatic microsomes: 20 mg/mL
(2) Test compound: 1.1 mM DMSO solution
(3) Potassium phosphate buffer solution: 66.7 mM (pH 7.4)
(4) NADPH solution: 10 mM in potassium phosphate buffer solution
(5) Quenching solution: 0.5% formic acid-acetonitrile solution with warfarin as internal standard substance

### 2. Method

Into a propylene tube, 971.5 µL of the potassium phosphate buffer solution and 27.5 µL of the mouse hepatic microsomes were put, and suspended. Thereto, 1 µL of a test compound was added, and 180 µL was taken from this mixture and transferred into another tube. The mixture was preincubated at 37°C for 5 minutes, and 20 µL of the NADPH solution (for the case with an incubation time of 30 minutes) or 20 µL of the potassium phosphate buffer solution (for the case with an incubation time of 0 minutes) was then added thereto. After incubation, 200 µL of the quenching solution was added to terminate the reaction. Subsequently, centrifugation was performed at 3220 ×g for 20 minutes, and the concentration of the unchanged form of the test compound in 200 µL of the supernatant was measured with LC-MS/MS. On the basis of the peak area obtained for the unchanged form, the residual rate (%) of the unchanged form was calculated as that for the case with an incubation time of 0 minutes was assumed as 100%.

### 3. Results

Table 3 shows residual rates (%) of unchanged forms after 30 minutes. It was confirmed that the compounds of Examples 1 to 9 are superior in metabolic stability in mouse hepatic microsomes to conventional ALDH2 activators.

### [Table 3]

**Table 3**

| | Residual rate (%) |
|---|---|
| Example 1 | 91 |
| Example 2 | 89 |
| Example 3 | 93 |
| Example 4 | 93 |
| Example 5 | 95 |
| Example 6 | >99 |
| Example 7 | 96 |
| Example 8 | 90 |
| Example 9 | 92 |
| Comparative Example 1 | <1 |
| Comparative Example 2 | 6 |
| Comparative Example 3 | 1 |
| Comparative Example 4 | <1 |
| Comparative Example 5 | <1 |
| Comparative Example 6 | 1 |

### [Test Example 3: Metabolic Stability with Human Hepatic Microsomes]

For the compounds of Examples 1 to 9, which had been confirmed to have stability in the mouse hepatic microsome test, metabolic stability with human hepatic microsomes was measured. The specific method is as follows.

(1) Fifty microliter-portions were taken from 0.2 µmol/L test compound diluted with β-NADPH solution.
(2) Fifty microliters of 0.2 mg protein/mL human hepatic microsome solution was added to each (except for a sample with a reaction time of 0 minutes).
(3) Incubation was performed with shaking at 37°C for 30 minutes (concentration in reaction sample: 0.1 µmol/L test compound, 0.1 mg protein/mL human hepatic microsome).
(4) After incubation, 400 µL of methanol was added to terminate the reaction.
(5) To the sample with a reaction time of 0 minutes, 50 µL of 0.2 mg protein/mL hepatic microsome solution was added.
(6) Each of the samples from (4) and (5) was left to stand at -20°C for 30 minutes or longer, and then centrifuged at 4°C and 3,000 rpm for approximately 10 minutes.
(7) The supernatant was subjected to measurement with LC/MS/MS, and, on the basis of the peak area obtained for the unchanged form, the residual rate (%) of the unchanged form was calculated as that for the case with an incubation time of 0 minutes was assumed as 100%.

Table 4 shows residual rates (%) of unchanged forms after 30 minutes.

### [Table 4]

**Table 4**

| | Residual rate (%) |
|---|---|
| Example 1 | 96 |
| Example 2 | >99 |
| Example 3 | 93 |
| Example 4 | >99 |
| Example 5 | 92 |
| Example 6 | 47 |
| Example 7 | 89 |
| Example 8 | >99 |
| Example 9 | 93 |

### [Test Example 4: Reactive Metabolite]

The risk of production of a reactive metabolite was examined for each test compound by measuring the production of a glutathione conjugate in the presence of human hepatic microsomes through LC-MS/MS. The specific method is as follows.

### 1. Method

(1) To a polypropylene tube, 55 µL of 20 mg/mL human hepatic microsome, 395 µL of 66.7 mM potassium phosphate buffer solution (pH 7.4), and 550 µL of 10 mM aqueous solution of glutathione were added.
(2) Added was 0.55 µL of 20 mM test compound or positive control (Clozapine, Reloxifen, Dicrofenac).
(3) A 180 µL-portion thereof was put in each of four tubes, with two of them labeled T0 and the other two labeled T60, and the tubes were preincubated at 37 ± 1°C for 5 minutes.
(4) To the T60 tubes and the T0 tubes, 20 µL of 10 mM NADPH solution and 20 µL of potassium phosphate buffer solution were added, respectively.
(5) After 60 minutes, 200 µL of 10% trichloroacetic acid-acetonitrile solution was added to each tube to terminate the reaction.
(6) Centrifugation was performed by using a centrifuge (5810-R, Eppendorf) at 3220 ×g for 20 minutes, and 200 µL of each supernatant was subjected to measurement of the GSH conjugate by LC-MS/MS (LC: SIL-HTc, Shimazu Corporation, Mass: API-4000 Qtrap, MDS Sciex).
(7) The ratio (%), area for GSH conjugate found for test compound/area for GSH conjugate found for clozapine, was calculated.

### 2. Results

Table 5 shows the results. These results confirmed that the compounds of Examples 1 to 9 cause less generation of the reactive metabolite than clozapine causes.

### [Table 5]

**Table 5**

| | Generation of reactive metabolite (% to clozapine) |
|---|---|
| Example 1 | <1 |
| Example 2 | <1 |
| Example 3 | 3 |
| Example 4 | <1 |
| Example 5 | <1 |
| Example 6 | <1 |
| Example 7 | <1 |
| Example 8 | <1 |
| Example 9 | <1 |

### [Test Example 5: Suppression Effect on Pain Induced by Carrageenan]

This experiment was carried out with reference to Science Translational Medicine 6, 251ra118 (2014). Animals used were 7-week-old male C57BL/6J mice (The Jackson Laboratory Japan, Inc.). To induce mechanical allodynia to a sole part, carrageenan was subcutaneously administered to the sole of the left hind limb of each mouse. The escape response against mechanical stimulation 180 minutes after the induction was measured by using a von Frey filament with bending force (0.16 grams) to the sole surface of the left hind limb in the ascending direction. The mouse was stimulated in the vertical direction for 6 seconds until the filament bent, and the escape response of the mouse was rated with three-grade scoring (0: no response or startle response (displacing the foot without ascending), 1: ascending the foot, 2: licking or shaking the foot). Stimulation was performed 10 times, and the total value of 10 scores (escape score) was calculated.

The compound (Example 3) was dissolved in DMSO/PEG400 (volume ratio: 1:1), and 2, 6, or 20 mg/kg (no compound for a Vehicle group) of the compound was subcutaneously administered to the back of the neck three times in total at the same dose: 15 minutes before injection of carrageenan, 30 minutes after injection of carrageenan, and 150 minutes after injection of carrageenan, each at a dose of 5 mL/kg in liquid volume. A 1.5% carrageenan solution was prepared with physiological saline, and subcutaneously administered to the sole of the left hind limb at 7 µL/body.

In significance test, multiple comparison by the Steel's test was carried out for comparison between the Vehicle group and Example 3 group, where the significance level was set to 5%, and calculation was separately performed for lower than 5% (p < 0.05) and for lower than 1% (p < 0.01). The commercially available statistical program SAS SYSTEM (SAS Institute Japan Ltd.) was used for the significance test.

As shown in Table 6, the compound according to the present invention was demonstrated to suppress pain induced by carrageenan.

### [Table 6]

**Table 6**

| Group | | Vehicle | Example 3 2 mg/kg | Example 3 6 mg/kg | Example 3 20 mg/kg |
|---|---|---|---|---|---|
| Number of animals | | 8 | 8 | 8 | 8 |
| Pain-related total score Time after induction with carrageenan (min) | | | | | |
| | Pre | 1.8±0.4 | 1.8±0.5 | 1.8±0.5 | 1.8±0.4 |
| | 180 | 9.4±0.5 | 5.3±0.3** | 4.6±0.3** | 3.9±0.3** |

| | | | | | |
|---|---|---|---|---|---|
| Each value represents mean ± SE. Pre: Before induction Significant difference from Vehicle group (*: p < 0.05, **: p < 0.01) | | | | | |

## Claims

1. A compound, a pharmaceutically acceptable salt of the compound, or a prodrug of the compound or the salt, the compound represented by the following formula (1): 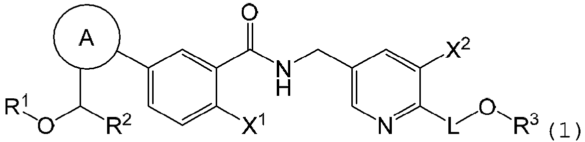 wherein
A is a heterocycle,
R¹ and R² are each independently hydrogen, an alkyl, an alkenyl, or an alkynyl,
R³ is an alkyl, an alkenyl, or an alkynyl,
L is absent or an alkylene,
X¹ is a halogen, and
X² is hydrogen or a halogen.

2. The compound, pharmaceutically acceptable salt of the compound, or prodrug of the compound or the salt according to claim 1, wherein A contains one or more nitrogen atoms as a ring member atom or ring member atoms.

3. The compound, pharmaceutically acceptable salt of the compound, or prodrug of the compound or the salt according to claim 1, wherein A is a five- or six-membered ring.

4. The compound, pharmaceutically acceptable salt of the compound, or prodrug of the compound or the salt according to claim 1, wherein A is an aromatic heterocycle.

5. The compound, pharmaceutically acceptable salt of the compound, or prodrug of the compound or the salt according to claim 1, wherein the compound represented by the formula (1) is a compound represented by the following formula (2): 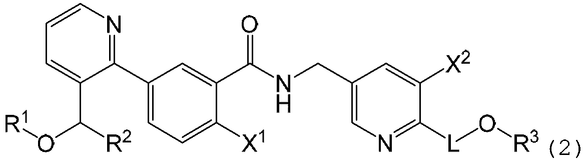 wherein R¹, R², R³, L, X¹, and X² are as described above.

6. The compound, pharmaceutically acceptable salt of the compound, or prodrug of the compound or the salt according to claim 1, wherein R¹ is hydrogen.

7. The compound, pharmaceutically acceptable salt of the compound, or prodrug of the compound or the salt according to claim 1, wherein R² is hydrogen or an alkyl.

8. The compound, pharmaceutically acceptable salt of the compound, or prodrug of the compound or the salt according to claim 1, wherein R³ is an alkyl.

9. The compound, pharmaceutically acceptable salt of the compound, or prodrug of the compound or the salt according to claim 1, wherein X¹ is fluorine.

10. The compound, pharmaceutically acceptable salt of the compound, or prodrug of the compound or the salt according to claim 1, wherein X² is hydrogen or fluorine.

11. The compound, pharmaceutically acceptable salt of the compound, or prodrug of the compound or the salt according to claim 1, wherein the compound represented by the formula (1) is selected from the group consisting of the following compounds:

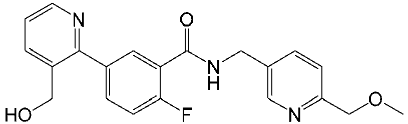

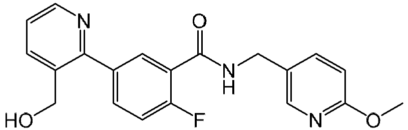

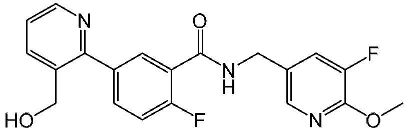

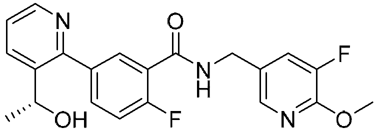

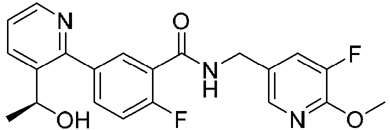

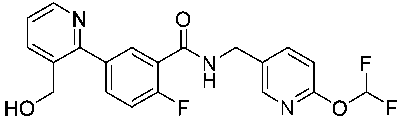

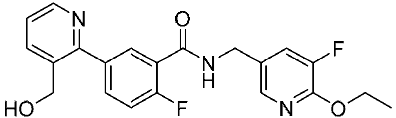

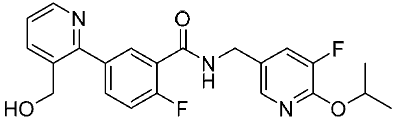

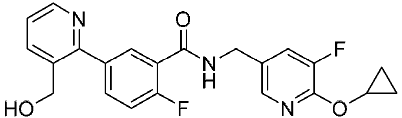

12. The prodrug according to claim 1 or a pharmaceutically acceptable salt of the prodrug, wherein R¹ is -CH₂-O-PO₃H₂.

13. The prodrug according to claim 1 or a pharmaceutically acceptable salt of the prodrug, wherein A contains one or more nitrogen atoms as a ring member atom or ring member atoms, and at least one of the nitrogen atoms is substituted with -CH₂-O-PO₃H₂.

14. An aldehyde dehydrogenase 2 activator comprising the compound, pharmaceutically acceptable salt of the compound, or prodrug of the compound or the salt according to any one of claims 1 to 13.

15. A pharmaceutical composition comprising the compound, pharmaceutically acceptable salt of the compound, or prodrug of the compound or the salt according to any one of claims 1 to 13.

16. A therapeutic and/or prophylactic drug comprising the compound, pharmaceutically acceptable salt of the compound, or prodrug of the compound or the salt according to any one of claims 1 to 13 for a disease selected from the group consisting of Fanconi's anemia, osteoporosis, nonalcoholic fatty liver disease (NAFLD), nonalcoholic steatohepatitis (NASH), alcoholic liver disease, pancreatitis, ischemia-reperfusion injury, peripheral arterial disease, Alzheimer's disease, Parkinson's disease, esophageal cancer, head and neck cancer, pain, and diabetic retinopathy.
